# EUROPEAN PATENT APPLICATION

(11) **EP 4 349 923 A1**
(43) Date of publication of application: **10.04.2024**
(21) Application number: 22815886.1
(22) Date of filing: 23.05.2022
(51) Int. Cl.: C09B 47/06, C09B 67/20

(54) **PHTHALOCYANINE COMPOUND**

(30) Priority: 31.05.2021 JP 2021091591
(71) Applicant: DIC Corporation, Tokyo 174-8520 (JP)
(72) Inventor: FOO Siongwan, Kamisu-shi Ibaraki 314-0193 (JP); EBATO Hiroshi, Tokyo 174-8520 (JP); SAKURAI Munenori, Tokyo 103-8233 (JP)
(74) Representative: TBK
(86) International application number: PCT/JP2022/021042
(87) International publication number: WO 2022/255135

(57) **Abstract**

Provided is a phthalocyanine compound in which the numbers and positions of various types of functional groups can be controlled and which can be produced from a biomass raw material, and thus, which has comparable or superior performance to conventional phthalocyanine compounds and can contribute to the carbon neutrality.

The phthalocyanine compound is a compound represented by the following formula (I) or the like, the compound containing a radioactive carbon atom ¹⁴C.

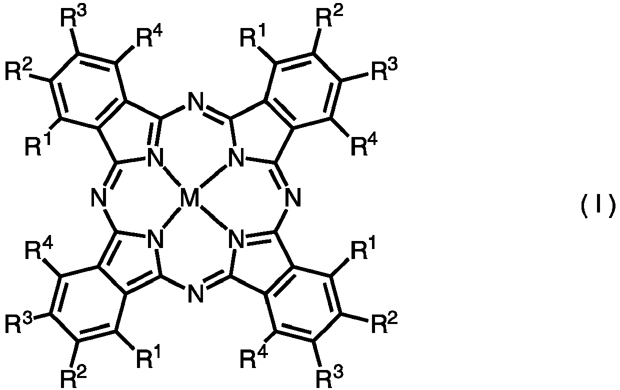

## Description

### TECHNICAL FIELD

The present invention relates to a phthalocyanine compound.

### BACKGROUND ART

Phthalocyanine is an excellent pigment that shows a hue of blue to green and is used for a wide range of applications. Phthalocyanine is currently generally synthesized from phthalic anhydride, phthalic acid, or phthalonitrile. There is also a synthetic approach from phthalimide or 1,3-diiminoisoindoline, but it is not commonly used. Since only limited types of phthalic acid derivatives can be used as a starting material of phthalocyanine synthesis, a method for derivatizing phthalocyanine which is the final product is also limited. Accordingly, in the industrial field, phthalocyanine having no functional group is generally produced.

The functional group that is bonded to a phthalocyanine backbone can finely control the hue depending on the electron donating capability and the electron withdrawing capability, and thus, is very important. Furthermore, a phthalocyanine having a functional group also acts as an additive that controls the crystal growth during the pigment formation. Due to the importance, methods for directly introducing a functional group into phthalocyanine have been developed. Examples of particularly successful cases include halogenation, sulfonation, and imidation of phthalocyanine (PTL 1, 2, and 3). However, in these methods, there are still remained problems in that the numbers and positions of functional groups cannot be controlled and the types of usable functional groups are also quietly limited. Accordingly, there is a need for development of a technique that can control the numbers and positions of functional groups of phthalocyanine and that can introduce a new functional group.

In addition, in recent years, from the viewpoints of saving oil resources and reducing carbon dioxide emission for sustainable development, an action for the carbon neutrality in which raw materials of chemical industrial products are switched from petroleum-derived raw materials to biomass raw materials has been activated. Thus, use of furfural (FF) and hydroxymethylfurfural (HMF) which are currently widely known to be producible from a (hemi)cellulose-derived sugar contained in inedible biomass has been studied (PTLs 4 and 5). The stream of conversion toward bio-renewable materials is also no exception in the field of organic pigments, but an organic pigment derived from a biomass raw material has not been achieved yet today.

### CITATION LIST

### PATENT LITERATURE

PTL 1: JP 2020-33526 A
PTL 2: JP 10-140025 A
PTL 3: JP 2016-23222 A
PTL 4: JP 5791838 B
PTL 5: JP 6328990 B

### SUMMARY OF INVENTION

### TECHNICAL PROBLEM

The problem to be solved by the invention is to provide a phthalocyanine compound in which the numbers and positions of various types of functional groups can be controlled and which can be produced from a biomass raw material, and thus, which has comparable or superior performance to conventional phthalocyanine compounds and can contribute to the carbon neutrality, and to provide a composition, a pigment composition, a printing ink, a printed article, a laminate of a printed article, a paint, a painted article, a coloring composition for a color filter, a color filter, and the like that are obtained by using the phthalocyanine compound.

### SOLUTION TO PROBLEM

As a result of intensive and extensive studies for solving the above problem, the present inventors have found that the above problem can be solved in such a manner that a biomass-derived furan derivative and maleic anhydride is used as raw materials, by a Diels-Alder (DA) reaction, a DA intermediate is obtained, the DA intermediate is subjected to ring opening dehydration to synthesize a phthalic anhydride derivative, and the phthalic anhydride derivative is used to perform phthalocyanine synthesis, whereby a phthalocyanine compound that contains a radioactive carbon atom ¹⁴C is produced, thus achieving completion of the invention.

Specifically, the invention includes the following aspects.
[1] A compound selected from a group of compounds represented by the following formulae (I), (II), (IA), (IIA), (IB), (IIB), (IC), (IIC), (ID), (IID), (IE), and (IIE), the compound containing a radioactive carbon atom ¹⁴C: 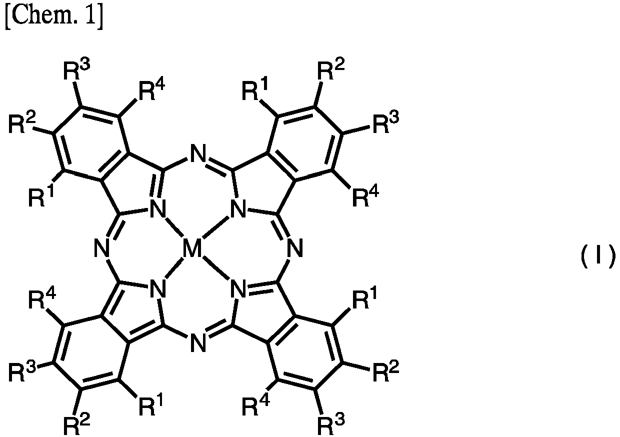 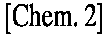 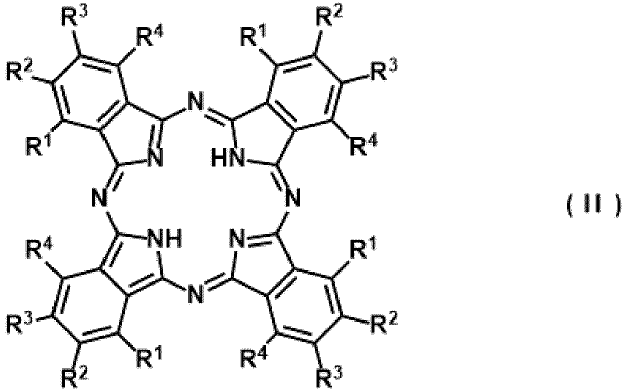 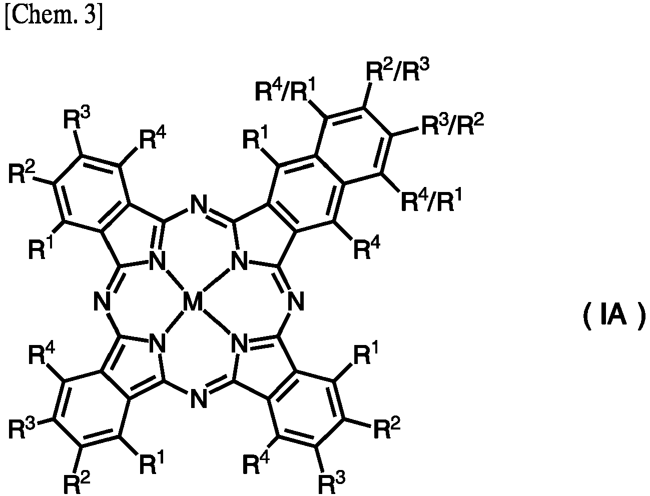 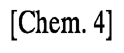 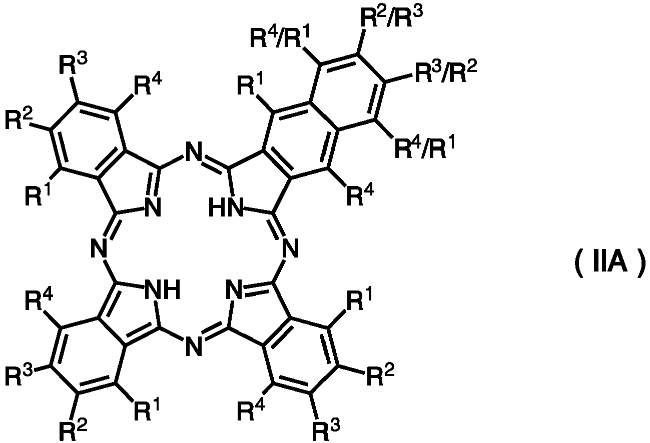 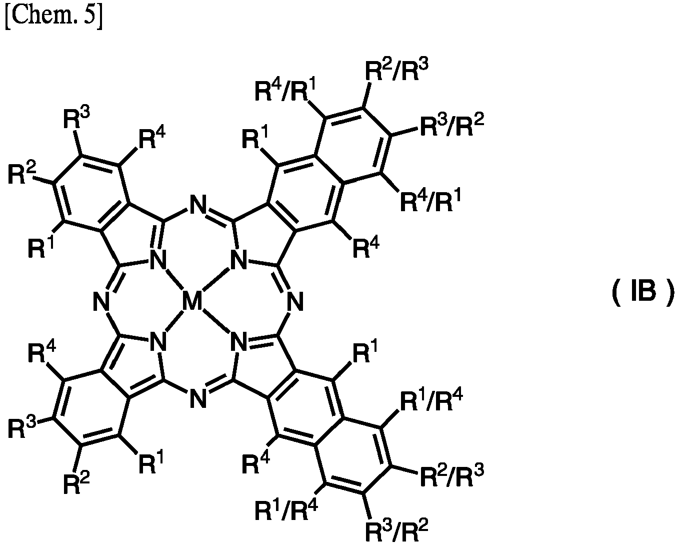 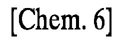 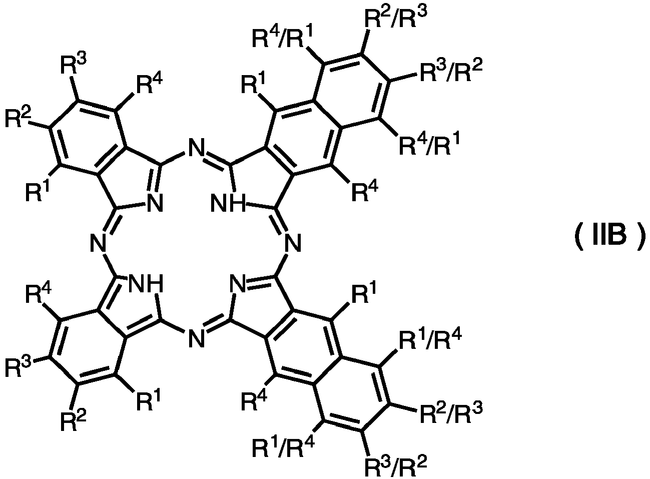 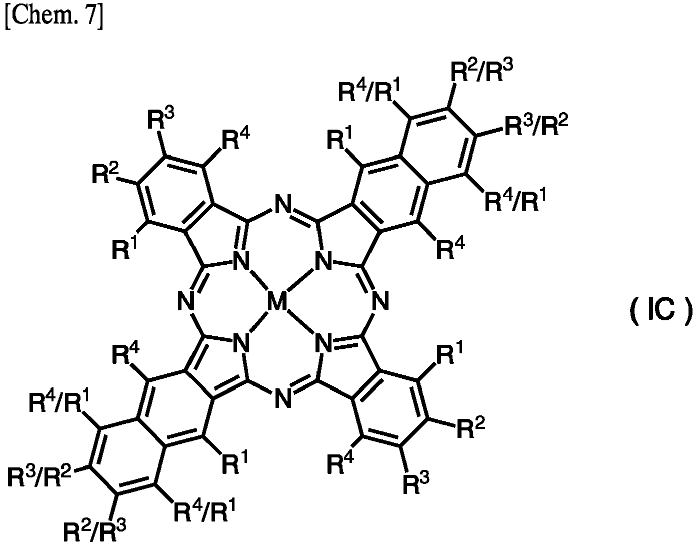 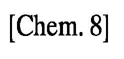 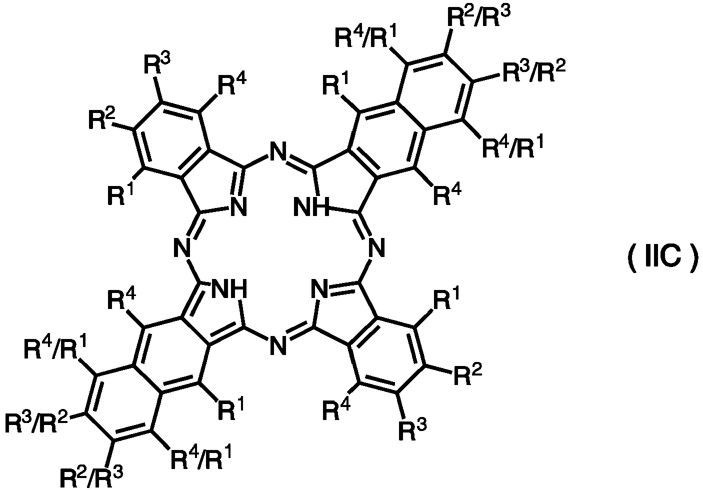 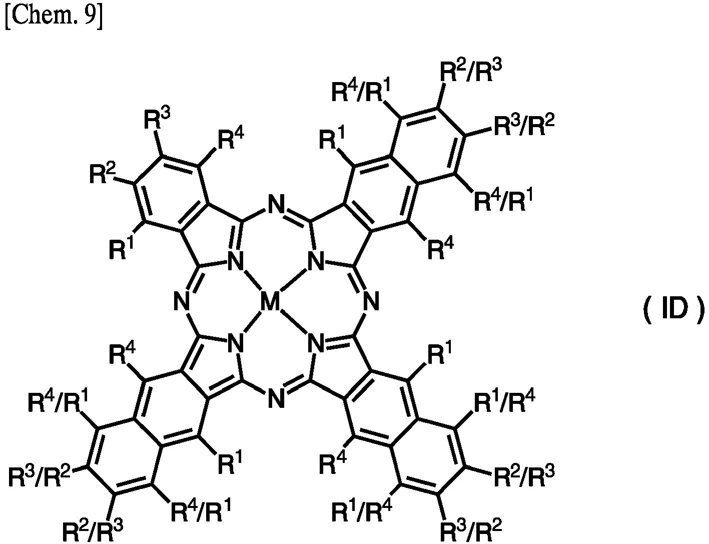 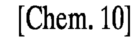 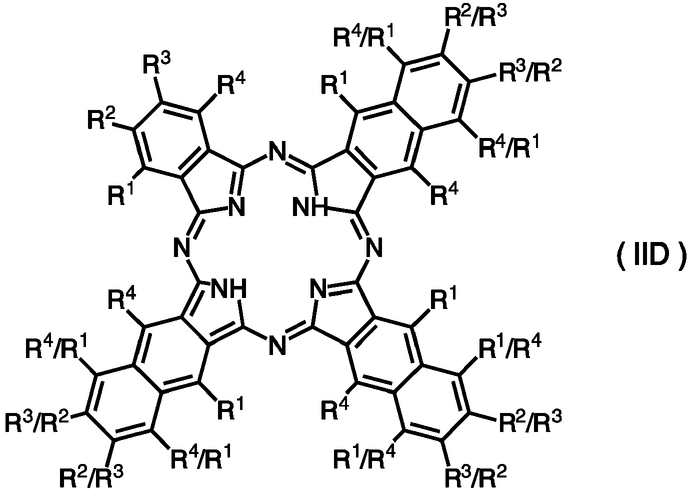 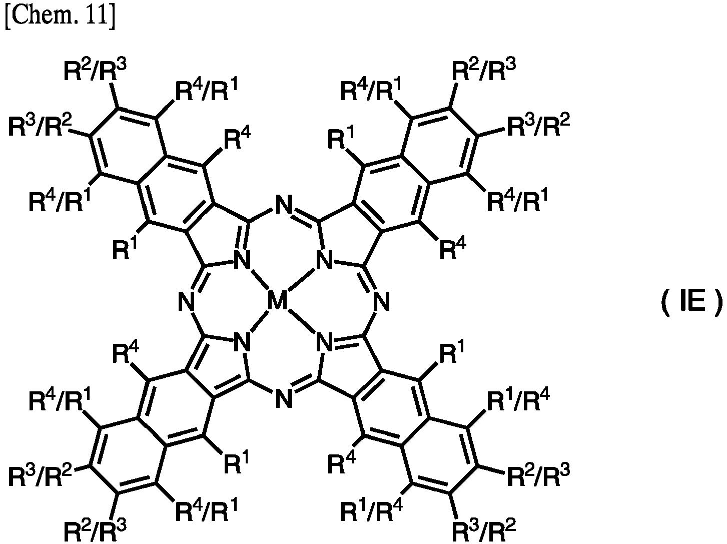 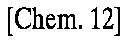 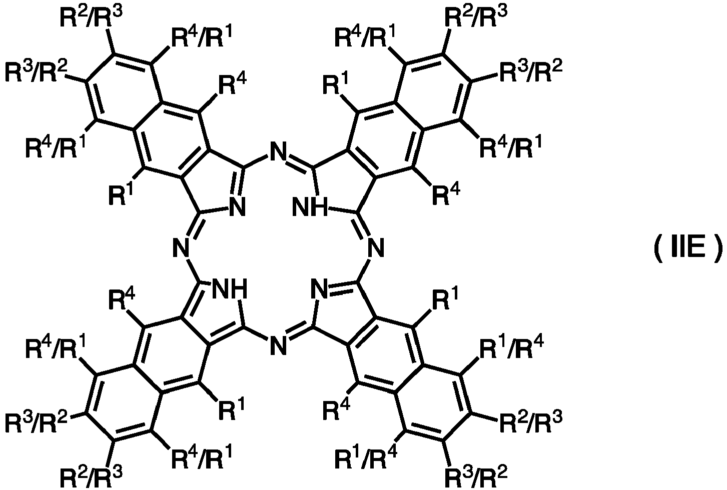 (in the formulae (I), (II), (IA), (IIA), (IB), (IIB), (IC), (IIC), (ID), (IID), (IE), and (IIE), R¹ to R⁴ each independently represent a hydrogen atom, a linear or branched alkyl group having 1 to 12 carbon atoms, a fluorine atom, a chlorine atom, a bromine atom, an iodine atom, a cyclohexyl group, or a phenyl group, one -CH₂- or non-adjacent two or more -CH₂- present in the alkyl group may be replaced by -C≡C-, -CH=CH-, -O-, -S-, -COO-, -OCO-, or -CO-, a hydrogen atom present in these groups may be substituted with a fluorine atom or a phenyl group, one -CH₂- or non-adjacent two or more -CH₂- present in the cyclohexyl group may be replaced by -O- or -S-, one -CH= or non-adjacent two or more -CH= present in the phenyl group may be replaced by -N=, one hydrogen atom or two or more hydrogen atoms present in this group may be substituted with a fluorine atom, and M represents a metal atom.)
[2] The compound according to [1], in which the metal atom represented by the above-mentioned M is one kind or two or more kinds selected from the group consisting of Al, Si, Sc, Ti, V, Mg, Fe, Co, Ni, Cu, Zn, Ga, Ge, Y, Zr, Nb, In, Sn, or Pb.
[3] The compound according to [1] or [2], in which the compound represented by (I), (II), (IA), (IIA), (IB), (IIB), (IC), (IIC), (ID), (IID), (IE), or (IIE) has pMC (percent modern carbon) of 20% or more.
[4] The compound according to any one of [1] to [3], in which the above-mentioned R¹ to R⁴ are all a hydrogen atom.
[5] A composition containing one compound or two or more compounds selected from the group of compounds represented by the formulae (I), (II), (IA), (IIA), (IB), (IIB), (IC), (IIC), (ID), (IID), (IE), and (IIE) according to [1].
[6] A pigment composition containing the compound according to [1] or the composition according to [5].
[7] A printing ink containing the pigment composition according to [6].
[8] A printed article having the printing ink according to [7] printed thereon.
[9] A laminate including the printed article according to [8].
[10] A paint containing the pigment composition according to [6].
[11] A painted article including a coating film formed of the paint according to [10].
[12] A coloring composition for a color filter, the coloring composition containing the pigment composition according to [6].
[13] A color filter containing the coloring composition for a color filter according to [12].

### ADVANTAGEOUS EFFECTS OF INVENTION

According to the invention, it is possible to provide a phthalocyanine compound in which the numbers and positions of various types of functional groups can be controlled and which can be produced from a biomass raw material, and thus, which has comparable or superior performance to conventional phthalocyanine compounds and can contribute to the carbon neutrality.

### BRIEF DESCRIPTION OF DRAWINGS

Fig. 1 shows a TEM observation result of a pigment of Comparative Example 1 before pigmentation.
Fig. 2 shows a TEM observation result of the pigment of Comparative Example 1 after pigmentation.
Fig. 3 shows a TEM observation result of a pigment of Example 1 before pigmentation.
Fig. 4 shows a TEM observation result of the pigment of Example 1 after pigmentation.
Fig. 5 shows a TEM observation result of a pigment of Example 2 before pigmentation.
Fig. 6 shows a TEM observation result of the pigment of Example 2 after pigmentation.

### DESCRIPTION OF EMBODIMENTS

The invention will be described in detail below. The description of the configuration requirements below is merely an example for explaining the invention, and the invention is not to be limited to the description.

### (Compound Containing Radioactive Carbon Atom ¹⁴C)

The compound of the invention contains a radioactive carbon atom ¹⁴C.

The compound of the invention is selected from a group of compounds represented by the following formulae (I), (II), (IA), (IIA), (IB), (IIB), (IC), (IIC), (ID), (IID), (IE), and (IIE) (hereinafter also referred to as the formulae (I) to (IIE)).

When the compound of the invention is applied to a printing ink, a paint, a colorant for a color filter, or the like, the compound is preferably provided in an aspect of a composition that contains one compound or two or more compounds selected from the group of compounds represented by the formulae (I) to (IIE). Above all, the compound is preferably provided in an aspect of a composition that contains one compound or two or more compounds selected from a group of compounds represented by compounds (I) and (II) and further contains one compound or two or more compounds selected from a group of compounds represented by the formulae (IA), (IIA), (IB), (IIB), (IC), (IIC), (ID), (IID), (IE), and (IIE) (hereinafter also referred to as the formulae (IA) to (IIE)).

### < Compounds Represented by Formulae (I), (II), (IA), (IIA), (IB), (IIB), (IC), (IIC), (ID), (IID), (IE), and (IIE) >

The compounds represented by the formulae (I) to (IIE) will be shown below.

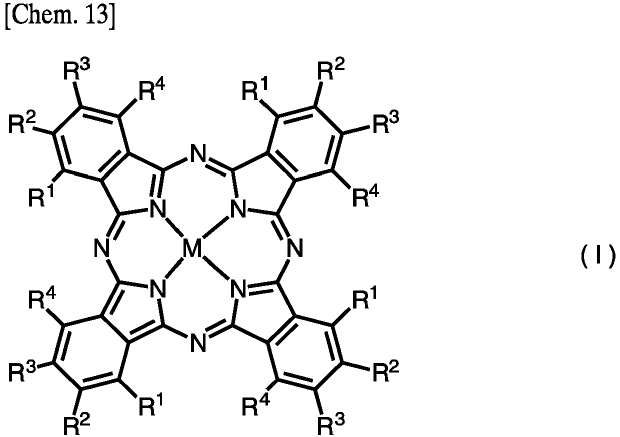

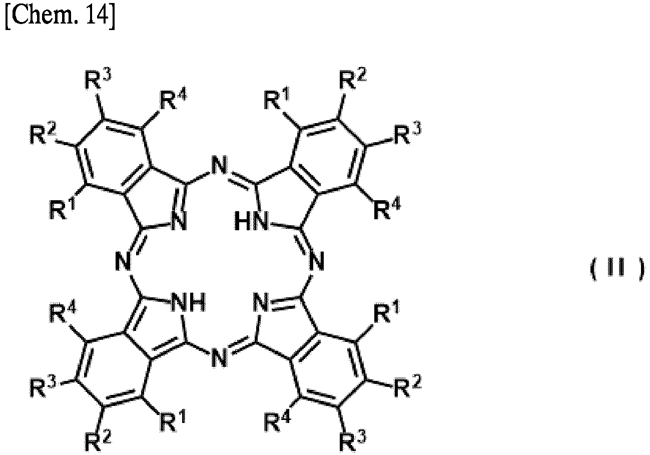

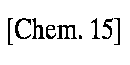

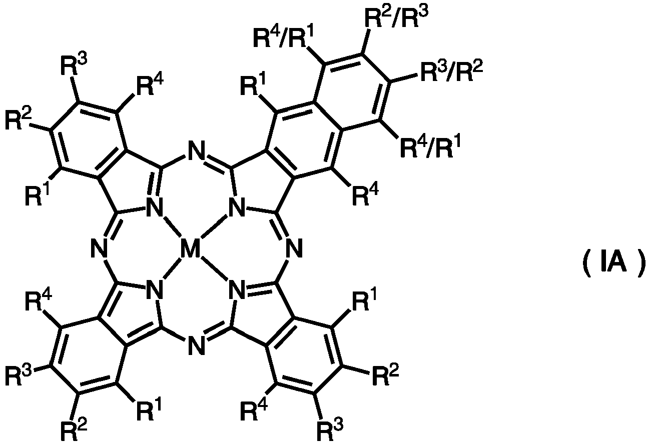

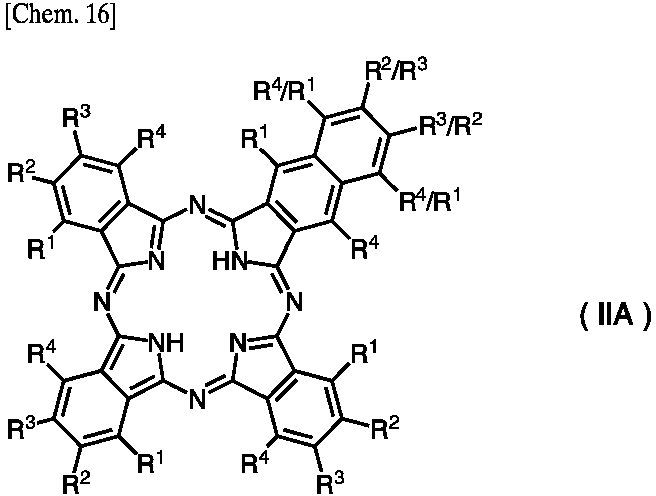

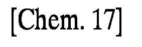

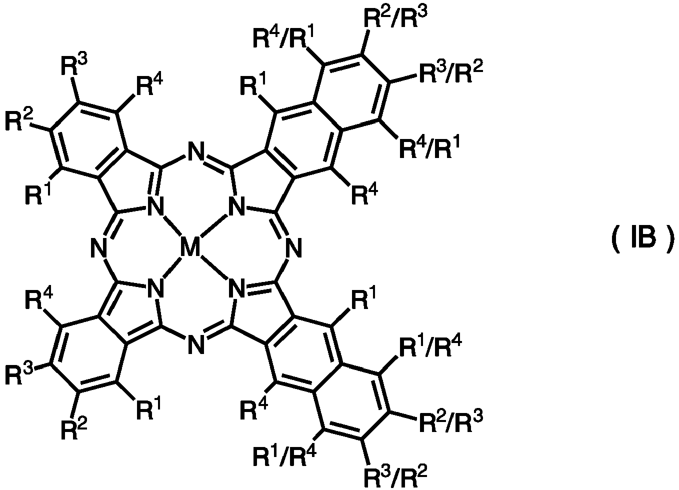

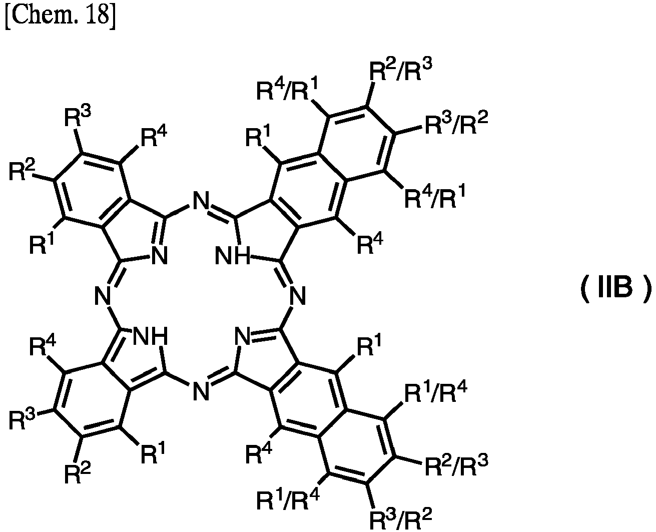

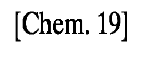

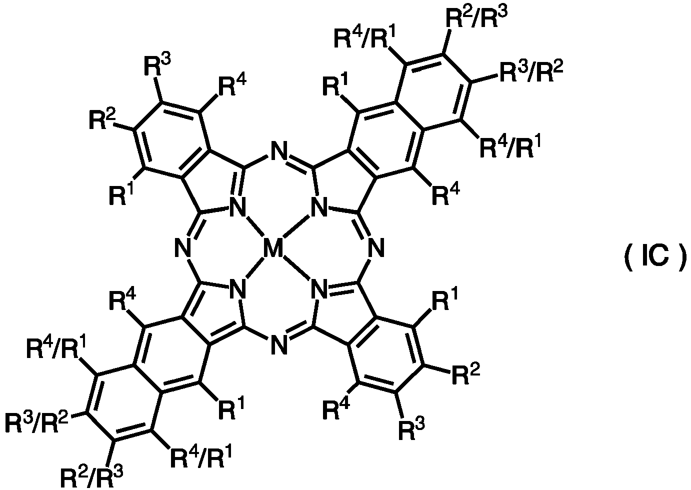

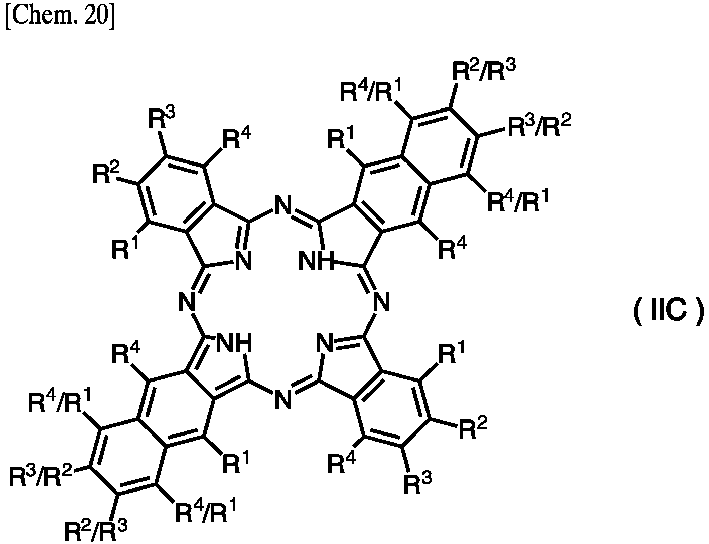

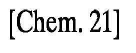

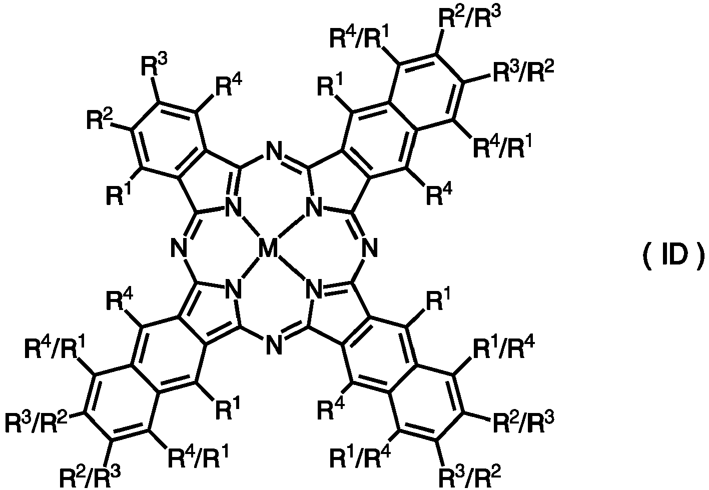

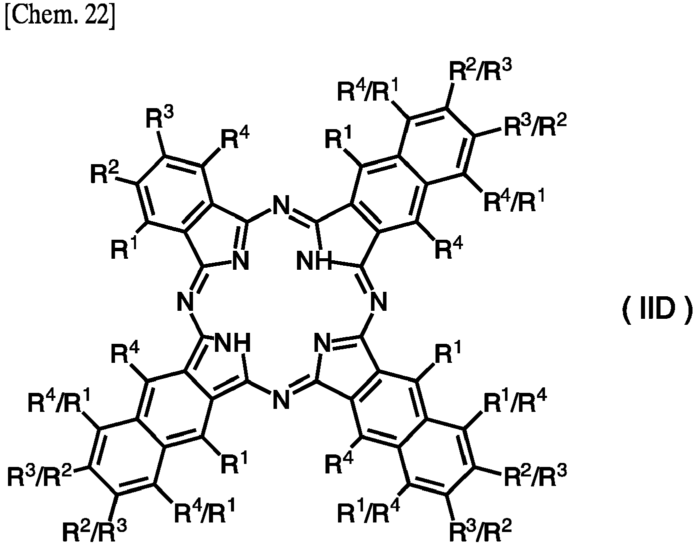

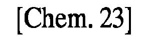

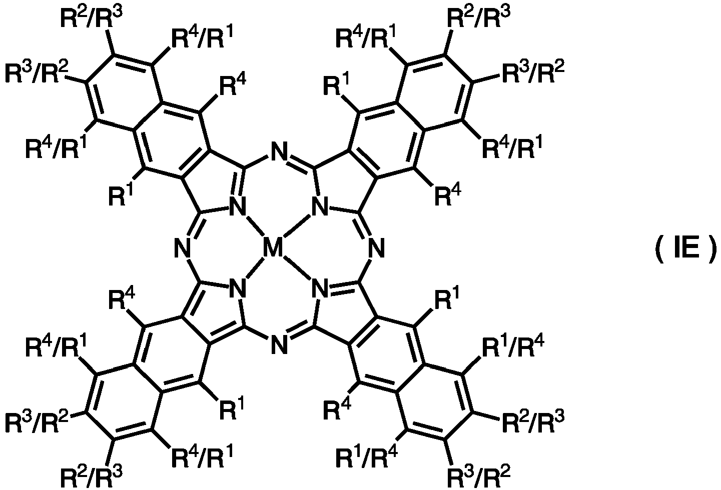

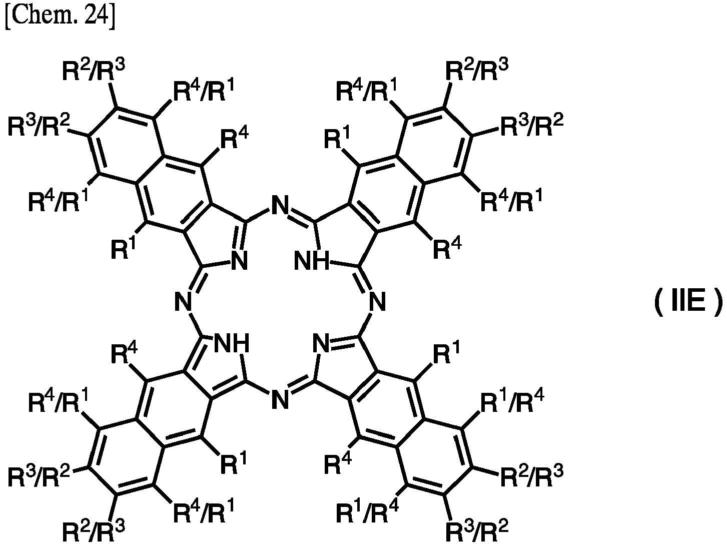

In the formulae (I) to (IIE), R¹ to R⁴ each independently represent a hydrogen atom, a linear or branched alkyl group having 1 to 12 carbon atoms, a fluorine atom, a chlorine atom, a bromine atom, an iodine atom, a cyclohexyl group, or a phenyl group, one-CH₂- or non-adjacent two or more -CH₂- present in the alkyl group may be replaced by-C≡C-, -CH=CH-, -O-, -S-, -COO-, -OCO-, or -CO-, a hydrogen atom present in these groups may be substituted with a fluorine atom or a phenyl group, one -CH₂- or non-adjacent two or more -CH₂- present in the cyclohexyl group may be replaced by -O- or -S-, one -CH= or non-adjacent two or more -CH= present in the phenyl group may be replaced by -N=, one hydrogen atom or two or more hydrogen atoms present in this group may be substituted with a fluorine atom, and M represents a metal atom.

Examples of the metal atom represented by M include one metal atom or two or more metal atoms selected from the group consisting of Al, Si, Sc, Ti, V, Mg, Fe, Co, Ni, Cu, Zn, Ga, Ge, Y, Zr, Nb, In, Sn, or Pb.

It is more preferred that R¹ to R⁴ in the formulae (I) to (IIE) are all a hydrogen atom.

The compounds represented by the formulae (I) to (IIE) preferably has pMC (percent modern carbon) of 20% or more, more preferably 40% or more, further preferably 60% or more, and particularly preferably 80% or more.

Here, the pMC (percent modern carbon) can be calculated by a measurement according to ASTM-D6866-18 and represents the percentage of the ¹⁴C concentration in an object relative to the ¹⁴C concentration in the standard modern carbon. In addition, when the object does not contain a radioactive carbon atom ¹⁴C, the pMC is 0%.

In the formulae (I) to (IIE), R¹ to R⁴ are preferably a hydrogen atom, a linear or branched alkyl group having 1 to 12 carbon atoms, a fluorine atom, a chlorine atom, or a bromine atom, more preferably a hydrogen atom, a linear or branched alkyl group having 1 to 12 carbon atoms, a chlorine atom, or a bromine atom, further preferably a hydrogen atom, a linear or branched alkyl group having 1 to 12 carbon atoms, or a bromine atom, and particularly preferably a hydrogen atom or a bromine atom.

In the formulae (I) to (IIE), M represents a metal atom, and is preferably Al, Si, Sc, Ti, V, Mg, Fe, Co, Ni, Cu, Zn, Ga, Ge, Y, Zr, Nb, In, Sn, or Pb, more preferably Al, Fe, Cu, or Zn, and further preferably Cu or Zn.

The biomass degree that the compound selected from the group of compounds represented by the formulae (I) to (IIE) shows is, for example, 1% or more, 5% or more, 10% or more, 15% or more, 20% or more, 25% or more, 30% or more, 35% or more, 40% or more, 45% or more, or 50% or more. Furthermore, the biomass degree is, for example, preferably 55% or more, more preferably 60% or more, more preferably 65% or more, more preferably 70% or more. In addition, the biomass degree is further preferably 75% or more, further preferably 80% or more, further preferably 85% or more. Moreover, the biomass degree is particularly preferably 90% or more, particularly preferably 95% or more.

When a biomass degree is within the above ranges, it results in containing biomass-derived carbon, and such a compound can contribute to reduction in the environmental load by the carbon neutrality.

Here, the biomass refers to a plant as an alternative energy source. The biomass is constituted mainly of two components, lignin and (hemi)cellulose. Lignin and (hemi)cellulose are both a polymer. Lignin is constituted of an aromatic monomer and (hemi)cellulose is constituted of a sugar having 5 carbon atoms and a sugar having 6 carbon atoms. In the compound described above, a lignin-derived raw material and a (hemi)cellulose-derived raw material can both be used as a raw material.

The biomass degree refers to the content (% by mass) of the biomass-derived carbon in the total carbon, calculated by a measurement according to ASTM-D6866-18.

Preferred embodiments of the compound represented by the formula (I) include, for example, compounds represented by the following formulae (I-1) to (I-12). 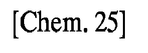 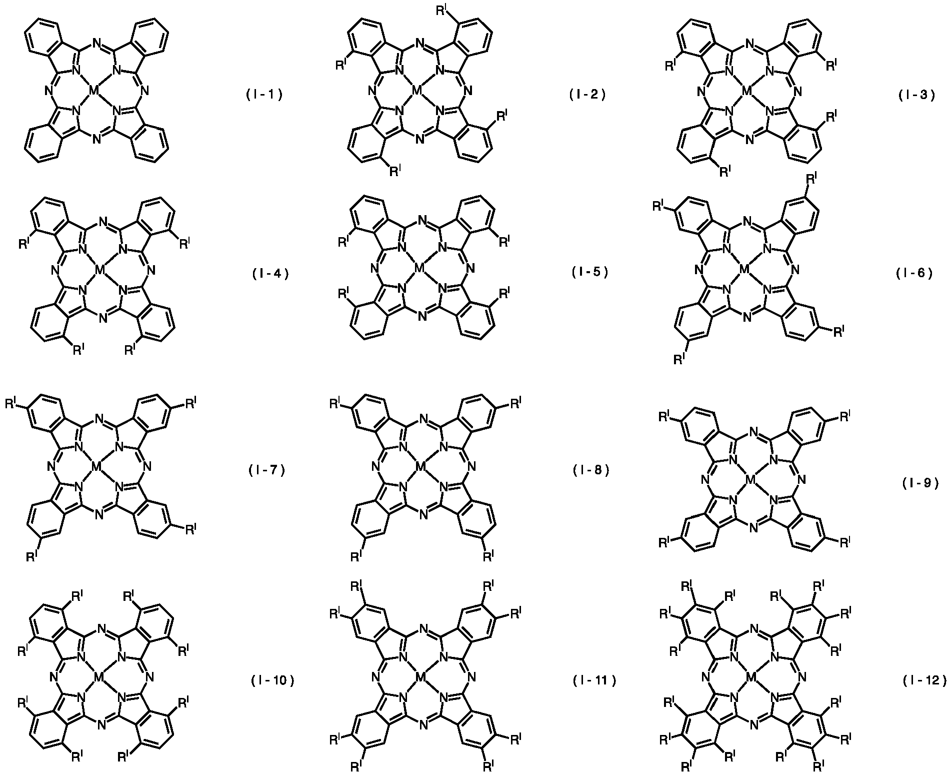 (In the formulae, R¹ represents the same meaning as R¹ to R⁴ described above, and M represents the same meaning as M described above.)

Examples of the compound represented by the formulae (I) include, but not limited to, compounds represented by the following formulae (I-1) to (I-12-2). 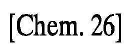 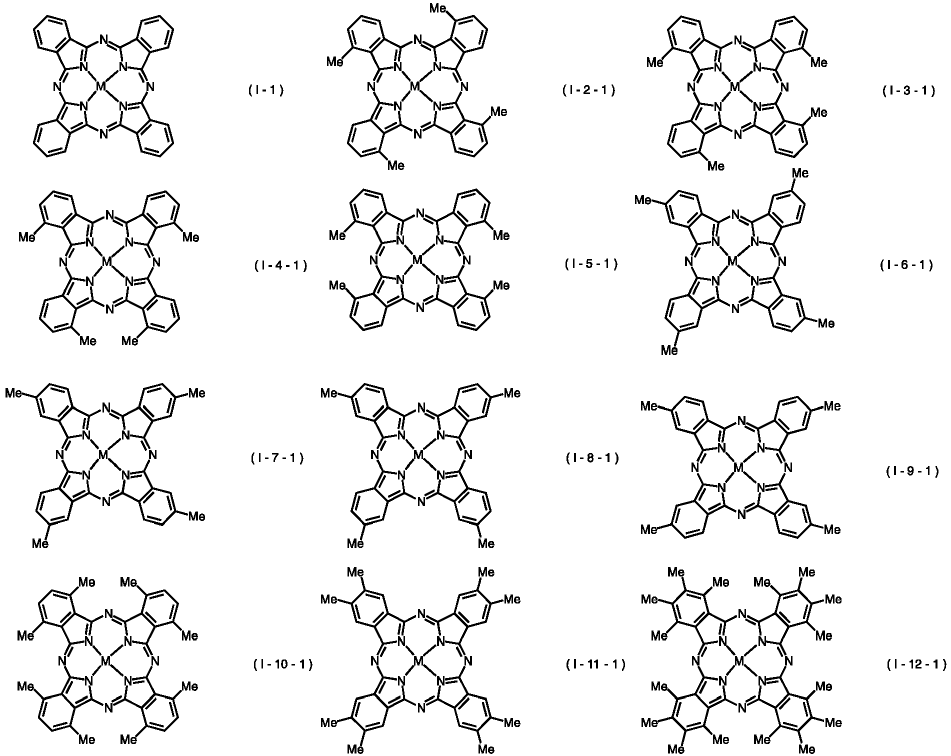 (In the formulae, M represents the same meaning as M described above.) 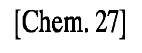 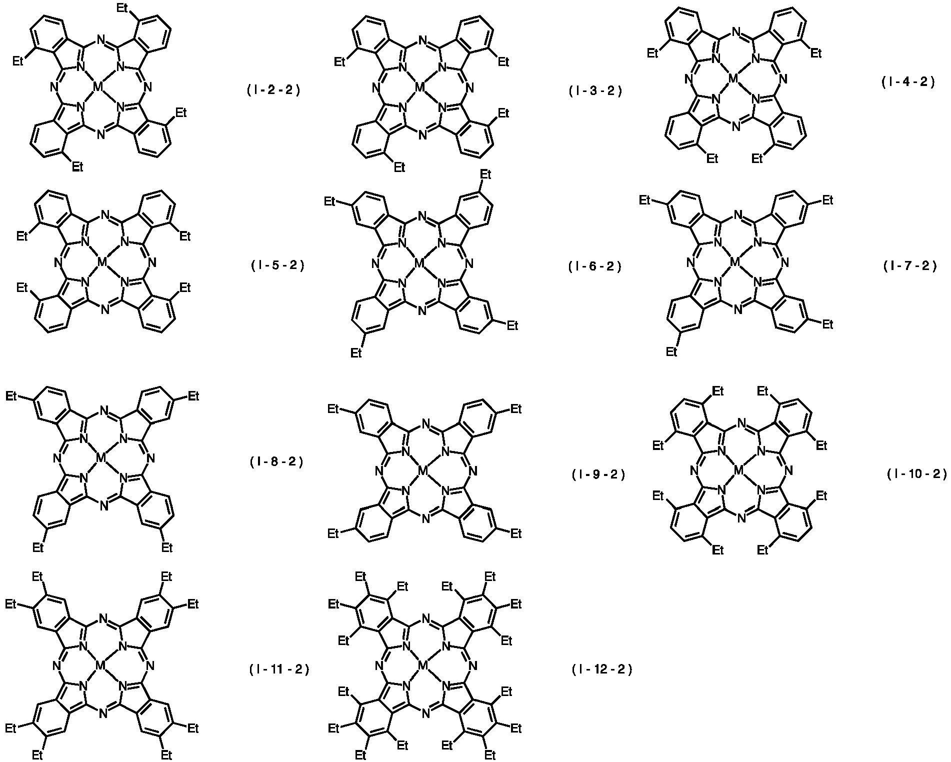 (In the formulae, M represents the same meaning as M described above.)

Preferred embodiments of the compound represented by the formulae (I-1) include compounds represented by the following formulae (I-1-1) to (I-1-4). 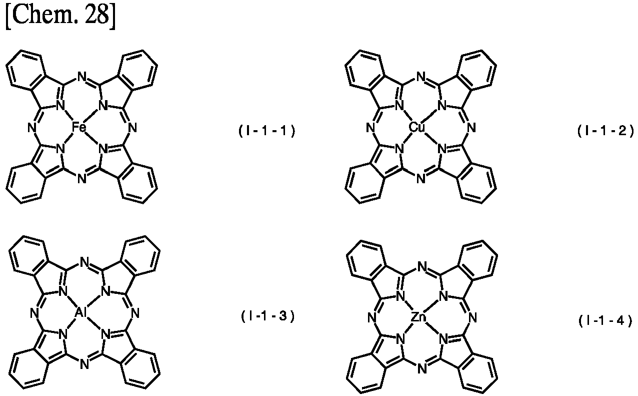 Among the compounds represented by the formulae (I-1-1) to (I-1-4), the compound represented by (I-1-2) is more preferred.

Preferred embodiments of the compound represented by the formulae (II) include compounds represented by the following formulae (II-1) to (II-12). 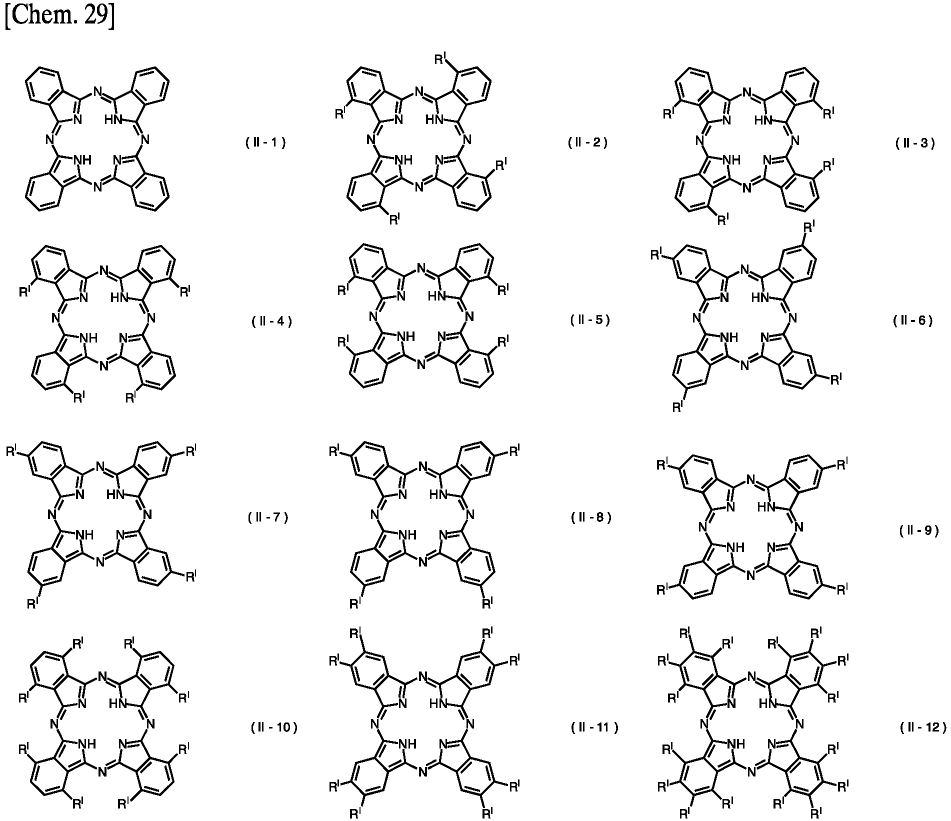 (In the formulae, R¹ represents the same meaning as R¹ to R⁴ described above and M represents the same meaning as M described above.)

Examples of the compound represented by the formula (II) include, but not limited to, compounds represented by the following formulae (II-1) to (II-12-2) and the like.

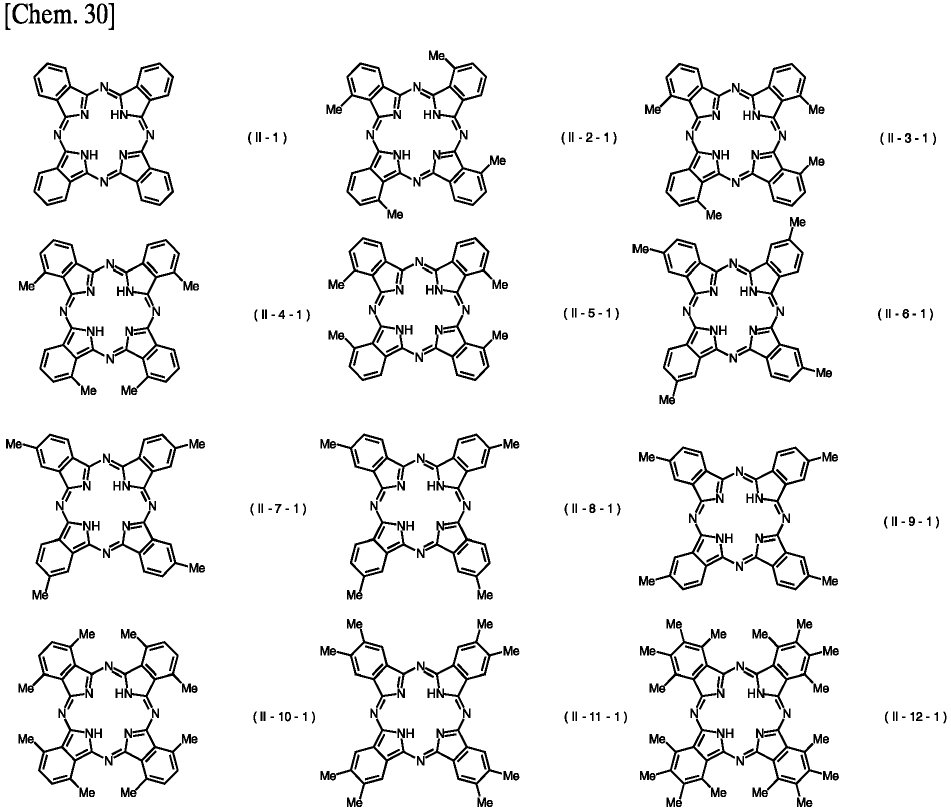

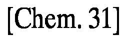

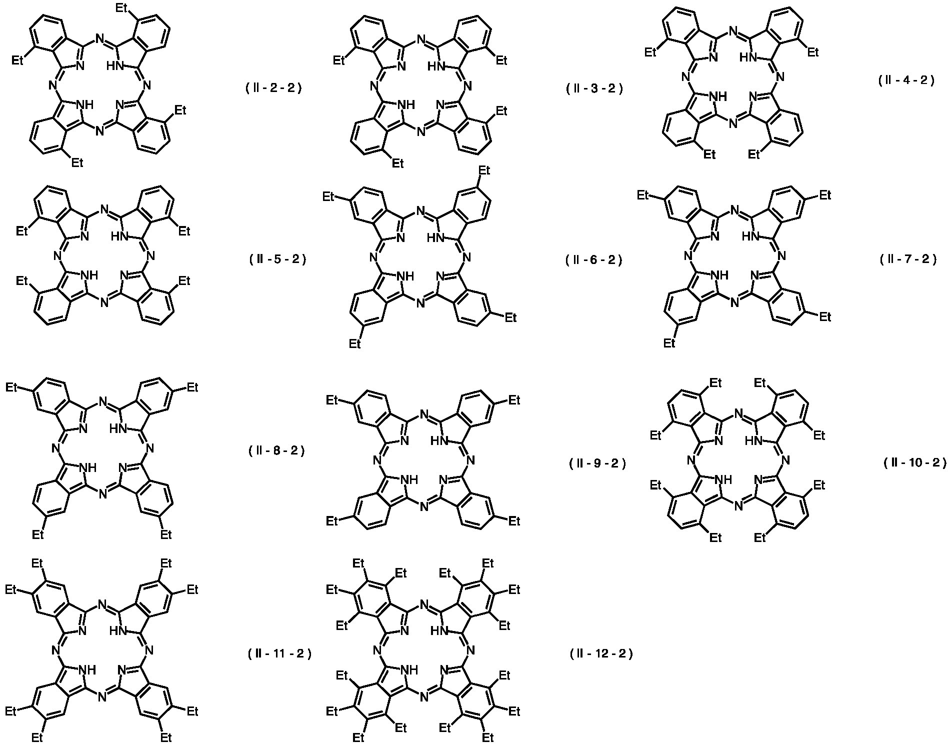

Among the compounds represented by the formulae (II-1) to (II-12), the compound represented by formula (II-1) is preferred.

Preferred aspects of the compounds represented by the formulae (IA), (IB), (IC), (ID), and (IE) include, for example, compounds represented by the following formulae (IA-1) to (IE-1).

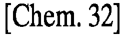

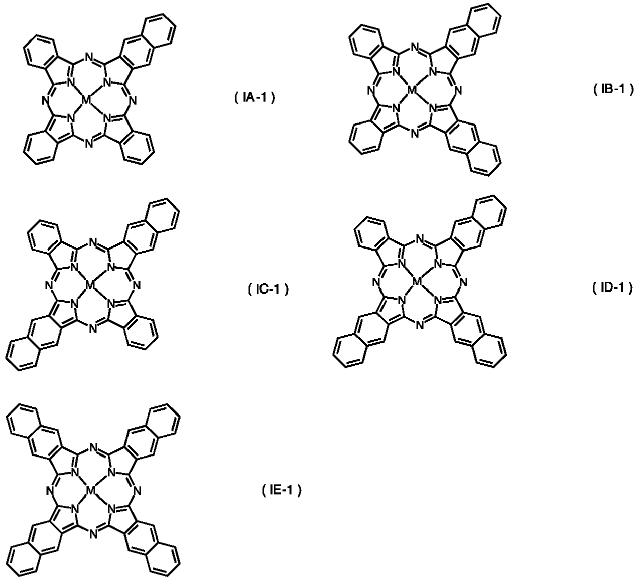

### (In the formulae, M represents the same meaning as M described above.)

As the compounds represented by the formulae (IA-1) to (IE-1), compounds represented by the following formulae (IA-1-1) to (IE-1-4) are preferred.

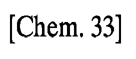

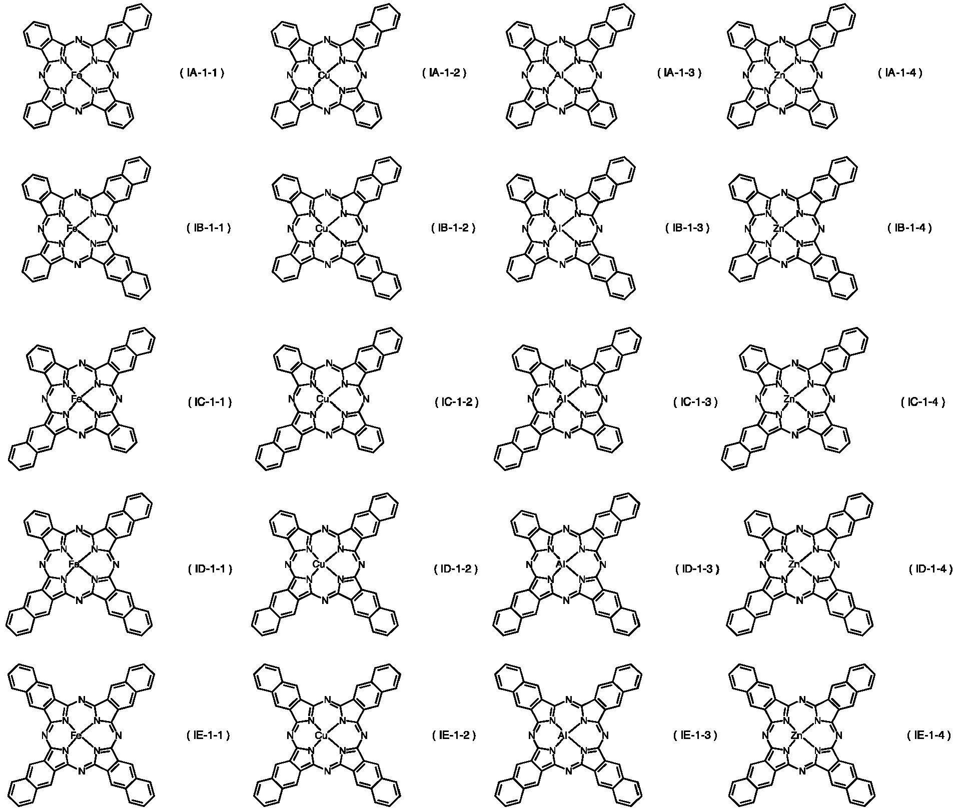

As the compounds represented by the formulae (IA-1-1) to (IE-1-4), the compounds represented by (IA-1-2), (IB-1-2), (IC-1-2), (ID-1-2), and (IE-1-2) are preferred. Among them, (IA-1-2) to (ID-1-2) are preferred, and (IA-1-2) to (IC-1-2) are more preferred, and (IA-1-2) is further preferred.

Preferred aspects of the compounds represented by the formulae (IIA), (IIB), (IIC), (IID), and (IIE) include, for example, compounds represented by the following formulae (IIA-1) to (IIE-1).

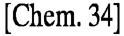

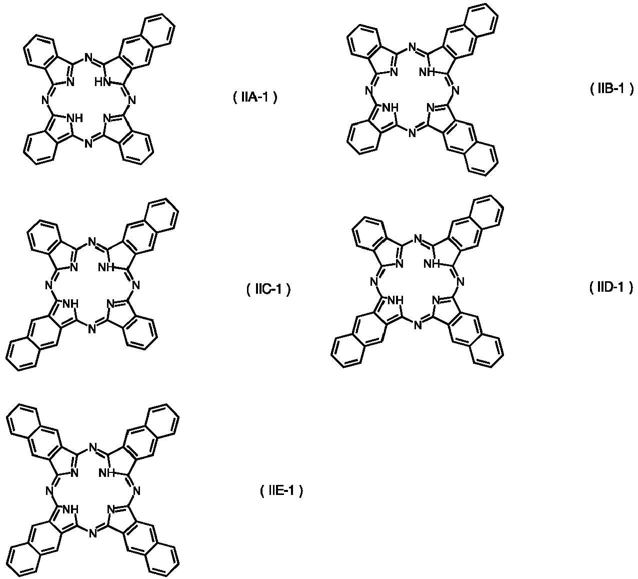

As the compounds represented by the formulae (IIA-1) to (IIE-1), the compounds represented by the formulae (IIA-1) to (IID-1) are preferred, the compounds represented by (IIA-1) to (IIC-1) are more preferred, and the compound represented by (IIA-1) is further preferred.

By the compounds represented by the formulae (IA) to (IIE), the pigment crystallization, the resin dispersibility, and the hue can be controlled, leading to high performance of a composition containing a compound selected from the group of compounds represented by the formulae (I) to (IIE) (hereinafter also referred to as phthalocyanine composition).

The compound selected from the group of compounds represented by the formulae (IA) to (IIE) is preferably a compound selected from a group of compounds represented by (IA) to (IID), more preferably a compound selected from a group of compounds represented by (IA) to (IIC), and further preferably a compound selected from a group of compounds represented by (IA) and (IIA).

The compound represented by the formula (I) or (II) (also referred to as Compound (I) or (II)) can be obtained by a method as described below.

### < Method for Producing Compound (I) or (II) >

A method for producing Compound (I) or (II) of the invention will be described below.

From a compound represented by the following formula (DA-1), a compound represented by the following formula (PA-1) is obtained, and from the compound represented by the formula (PA-1), the compound represented by the formula (I) or (II) of the invention can be obtained. 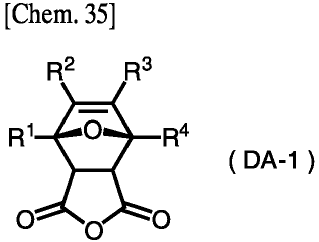 (In the formula, R¹ to R⁴ each independently represent a hydrogen atom, a linear or branched alkyl group having 1 to 12 carbon atoms, a fluorine atom, a chlorine atom, a bromine atom, an iodine atom, a cyclohexyl group, or a phenyl group, one -CH₂- or non-adjacent two or more-CH₂- present in the alkyl group may be replaced by -C≡C-, -CH=CH-, -O-, -S-, -COO-,-OCO-, or -CO-, a hydrogen atom present in these groups may be substituted with a fluorine atom or a phenyl group, one -CH₂- or non-adjacent two or more -CH₂- present in the cyclohexyl group may be replaced by -O- or -S-, one -CH= or non-adjacent two or more-CH= present in the phenyl group may be replaced by -N=, and one hydrogen atom or two or more hydrogen atoms present in this group may be substituted with a fluorine atom.) 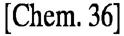 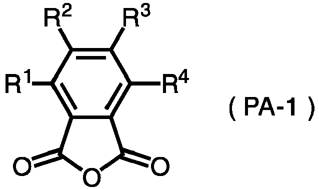 (In the formula, R¹ to R⁴ represent the same meaning as R¹ to R⁴ described above.)

Examples of the compound represented by the formula (DA-1) include, but not limited to, the following Compounds (DA-1-1) to (DA-1-17).

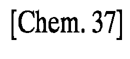

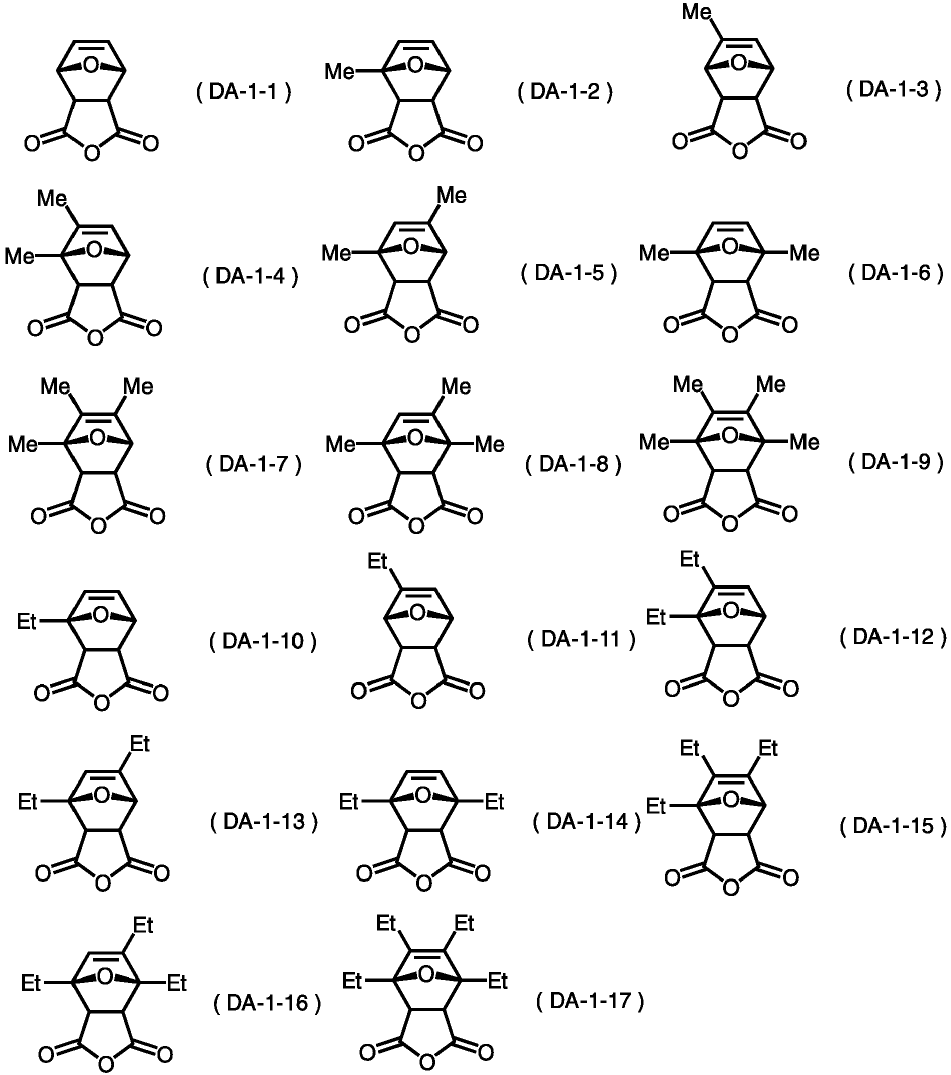

Examples of the compound represented by the formula (PA-1) include, but not limited to, the following Compounds (PA-1-1) to (PA-1-17).

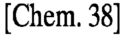

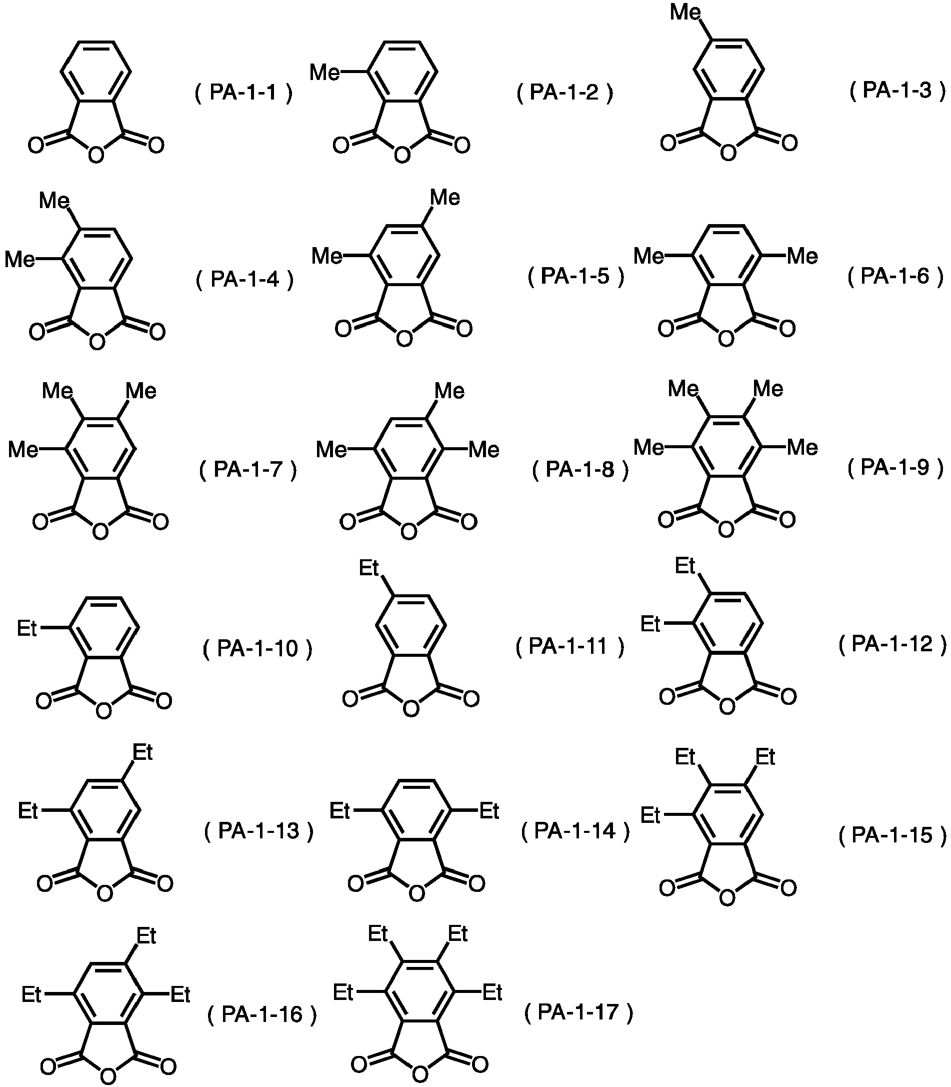

Furthermore, the compound represented by the formula (DA-1) may be any as long as the reaction suitably proceeds, but is preferably obtained by allowing a compound represented by the following formula (FR-1) to react with maleic anhydride. 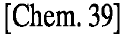 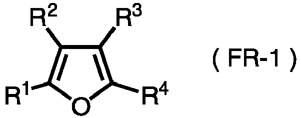 (In the formula, R¹ to R⁴ represent the same meaning as R¹ to R⁴ described above.)

Examples of the compound represented by the formula (FR-1) include, but not limited to, the following Compounds (FR-1-1) to (FR-1-5).

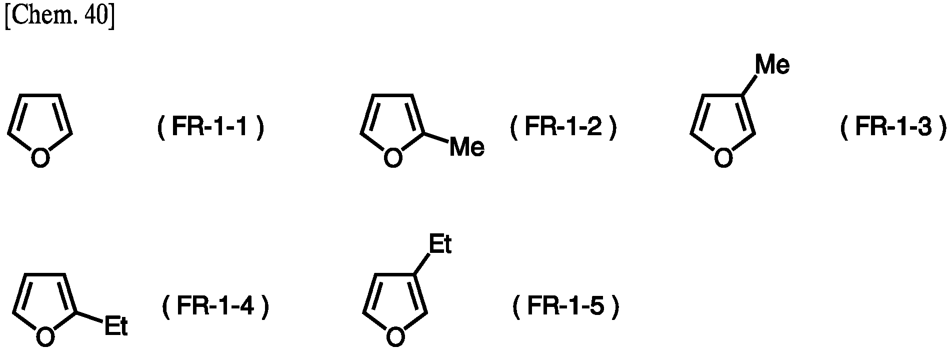

When the compound represented by the formula (PA-1) is obtained from the compound represented by the formula (DA-1), a catalyst, which may be any as long as the reaction suitably proceeds, is preferably used.

The catalyst may be any as long as the reaction suitably proceeds, but is preferably hydrochloric acid, sulfuric acid, p-toluenesulfonic acid, methanesulfonic acid, trifluoromethanesulfonic acid, polyphosphoric acid, diphosphoric acid, trifluoroacetic acid, or a compound represented by the following formula (AC). 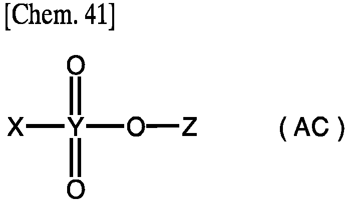 (In the formula, X represents -OH, -ONa, -OK, or -R⁵, R⁵ represents the same meaning as R¹ to R⁴ described above, Y represents a sulfur atom or a phosphorus atom, Z represents a hydrogen atom, -COR⁶, -COH, or -CO-CF₃, and R⁶ represents the same meaning as R¹ to R⁴ described above.)

The amount of the catalyst relative to the compound represented by the formulae (DA-1) is preferably 0.1 to 3000% by mole, preferably 0.5 to 2500% by mole, preferably 1 to 2000% by mole, preferably 5 to 1500% by mole, preferably 10 to 1000% by mole, preferably 20 to 500% by mole, preferably 50 to 500% by mole, preferably 70 to 500% by mole, preferably 100 to 500% by mole, preferably 150 to 500% by mole, preferably 200 to 500% by mole, preferably 250 to 500% by mole, preferably 300 to 500% by mole. The lower limit is preferably 0.1% by mole or more, preferably 0.5% by mole or more, preferably 1% by mole or more, preferably 5% by mole or more, preferably 10% by mole, preferably 20% by mole or more, preferably 50% by mole or more, preferably 70% by mole or more, preferably 100% by mole or more, preferably 150% by mole or more, preferably 200% by mole or more, preferably 250% by mole or more, preferably 300% by mole or more. The upper limit is preferably 3000% by mole or less, preferably 2500% by mole or less, preferably 2000% by mole or less, preferably 1500% by mole or less, preferably 1000% by mole or less, preferably 500% by mole or less. The upper limits and the lower limits may be used in any combination.

In this production method, all of R¹ to R⁴ preferably represent a hydrogen atom.

The maleic anhydride is preferably derived from biomass.

The compound represented by the formula (FR-1) is preferably derived from biomass.

It is more preferred that both the maleic anhydride and the compound represented by the formula (FR-1) are derived from biomass.

Raw materials used in the production method have a biomass degree of preferably 1% or more, preferably 5% or more, preferably 10% or more, preferably 15% or more, preferably 20% or more, preferably 25% or more, preferably 30% or more, preferably 35% or more, preferably 40% or more, preferably 45% or more, preferably 50% or more, preferably 55% or more, preferably 60% or more, preferably 65% or more, preferably 70% or more, preferably 75% or more, preferably 80% or more, more preferably 85% or more, further preferably 90% or more, and particularly preferably 95% or more.

The biomass-derived maleic anhydride can be obtained, for example, by cyclodehydration of maleic acid which is obtained by oxidating FF or HMF obtained by the method described in PTL 4 or 5, or can also be obtained by direct oxidation thereof. The biomass-derived compound represented by the formula (FR-1) can be obtained, for example, by subjecting FF or HMF obtained by the method described in PTL 4 or 5 to a proper combination of a decarbonylation reaction, a reduction reaction, and a dehydration reaction.

A specific reaction route of the production method is shown below.

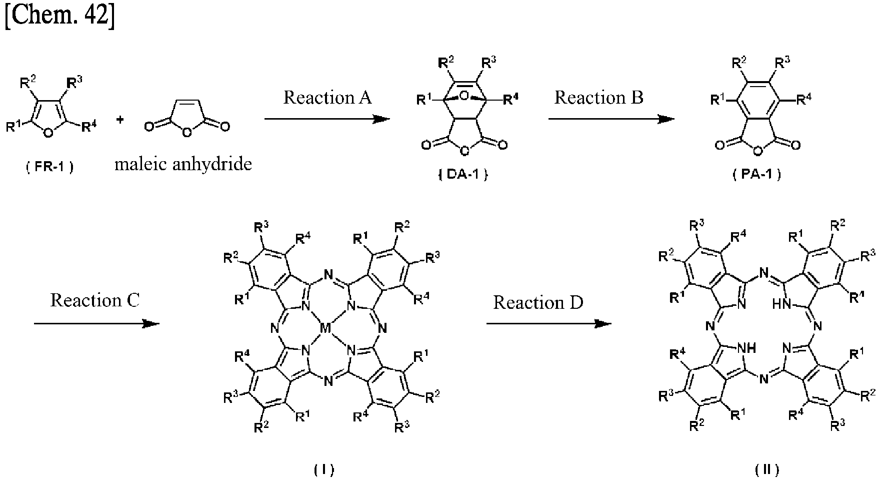

### < < Reaction A> >

Compound (DA-1) can be produced by subjecting Compound (FR-1) and maleic anhydride to a Diels-Alder reaction.

The reaction solvent may be any as long as the reaction suitably proceeds, but is preferably chloroform, dioxane, ethyl acetate, an alkylbenzene, toluene, xylene, or diethyl ether.

The reaction temperature may be any as long as the reaction suitably proceeds, but is preferably -10 to 100°C, more preferably 0°C to 80°C, further preferably 10°C to 70°C, and particularly preferably 15°C to 50°C. The lower limit is preferably -10°C or higher, more preferably 0°C or higher, further preferably 10°C or higher, and particularly preferably 15°C or higher. The upper limit is preferably 100°C or lower, more preferably 80°C or lower, further preferably 70°C or lower, and particularly preferably 50°C or lower.

The reaction pressure may be any as long as the reaction suitably proceeds, but is preferably 0.1 to 5 MPa, more preferably 0.1 to 3 MPa, further preferably 0.1 to 1 MPa, and particularly preferably 0.1 to 0.5 MPa. The lower limit is preferably 0.1 MPa or more, preferably 0.2 MPa or more, preferably 0.3 MPa or more, preferably 0.4 MPa or more. The upper limit is preferably 5 MPa or less, preferably 3 MPa or less, preferably 1 MPa or less, preferably 0.9 MPa or less, preferably 0.8 MPa or less, preferably 0.7 MPa or less, preferably 0.6 MPa or less, preferably 0.5 MPa or less.

### < <Reaction B> >

Compound (PA-1) can be produced by subjecting Compound (DA-1) obtained in Reaction A to a ring-opening dehydration reaction.

The reaction solvent may be any as long as the reaction suitably proceeds, but water, acetonitrile, toluene, xylene, an alkylbenzene, a mixed solvent thereof, or no solvent is preferably used.

The reaction temperature may be any as long as the reaction suitably proceeds, but is preferably 20 to 150°C, more preferably 30 to 120°C, more preferably 40 to 100°C. The lower limit is preferably 20°C or higher, preferably 25°C or higher, preferably 30°C or higher, preferably 35°C or higher, preferably 40°C or higher. The upper limit is preferably 150°C or lower, preferably 140°C or lower, preferably 130°C or lower, preferably 120°C or lower, preferably 110°C or lower, preferably 100°C or lower.

In the reaction, a catalyst is preferably used. The catalyst may be any as long as the reaction suitably proceeds, but is preferably hydrochloric acid, sulfuric acid, p-toluenesulfonic acid, methanesulfonic acid, trifluoromethanesulfonic acid, polyphosphoric acid, diphosphoric acid, trifluoroacetic acid, or a compound represented by the following formula (AC). 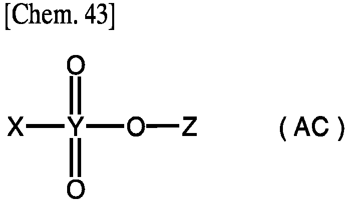 (In the formula, X represents -OH, -ONa, -OK, or -R⁵, R⁵ represents the same meaning as R¹ to R⁴ described above, Y represents a sulfur atom or a phosphorus atom, Z represents a hydrogen atom, -COR⁶, -COH, or -CO-CF₃, and R⁶ represents the same meaning as R¹ to R⁴ described above.)

The amount of the catalyst relative to the compound represented by the formula (DA-1) is preferably 0.1 to 3000% by mole, preferably 0.5 to 2500% by mole, preferably 1 to 2000% by mole, preferably 5 to 1500% by mole, preferably 10 to 1000% by mole, preferably 20 to 500% by mole, preferably 50 to 500% by mole, preferably 70 to 500% by mole, preferably 100 to 500% by mole, preferably 150 to 500% by mole, preferably 200 to 500% by mole, preferably 250 to 500% by mole, preferably 300 to 500% by mole. The lower limit is preferably 0.1% by mole or more, preferably 0.5% by mole or more, preferably 1% by mole or more, preferably 5% by mole or more, preferably 10% by mole, preferably 20% by mole or more, preferably 50% by mole or more, preferably 70% by mole or more, preferably 100% by mole or more, preferably 150% by mole or more, preferably 200% by mole or more, preferably 250% by mole or more, preferably 300% by mole or more. The upper limit is 3000% by mole or less, preferably 2500% by mole or less, preferably 2000% by mole or less, preferably 1500% by mole or less, preferably 1000% by mole or less, preferably 500% by mole or less. The upper limits and the lower limits may be used in any combination.

### < <Reaction C> >

Compound (I) can be produced by allowing Compound (PA-1) obtained by Reaction B to react with urea and MX in the presence of a catalyst.

The reaction solvent may be any as long as the reaction suitably proceeds, but no solvent or an alkylbenzene is preferably used.

The reaction temperature may be any as long as the reaction suitably proceeds, but is preferably 100 to 250°C, preferably 110 to 240°C, preferably 120 to 230°C, preferably 130 to 220°C, preferably 140 to 210°C, preferably 150 to 200°C. The lower limit is preferably 100°C or higher, preferably 110°C or higher, preferably 120°C or higher, preferably 130°C or higher, preferably 140°C or higher, preferably 150°C or higher. The upper limit is preferably 250°C or lower, preferably 240°C or lower, preferably 230°C or lower, preferably 220°C or lower, preferably 210°C or lower, preferably 200°C or lower.

M in the MX represents a metal atom, and is preferably Al, Si, Sc, Ti, V, Mg, Fe, Co, Ni, Cu, Zn, Ga, Ge, Y, Zr, Nb, In, Sn, or Pb, more preferably Al, Fe, Cu, or Zn, and further preferably Cu or Zn.

X in the MX represents a halogen atom, and is more preferably a chlorine atom.

The catalyst may be any as long as the reaction suitably proceeds, but is preferably a molybdenum catalyst, and more preferably ammonium molybdate tetrahydrate.

By this reaction, the compounds represented by the following (i-1) to (i-5) can also be produced. 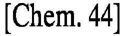 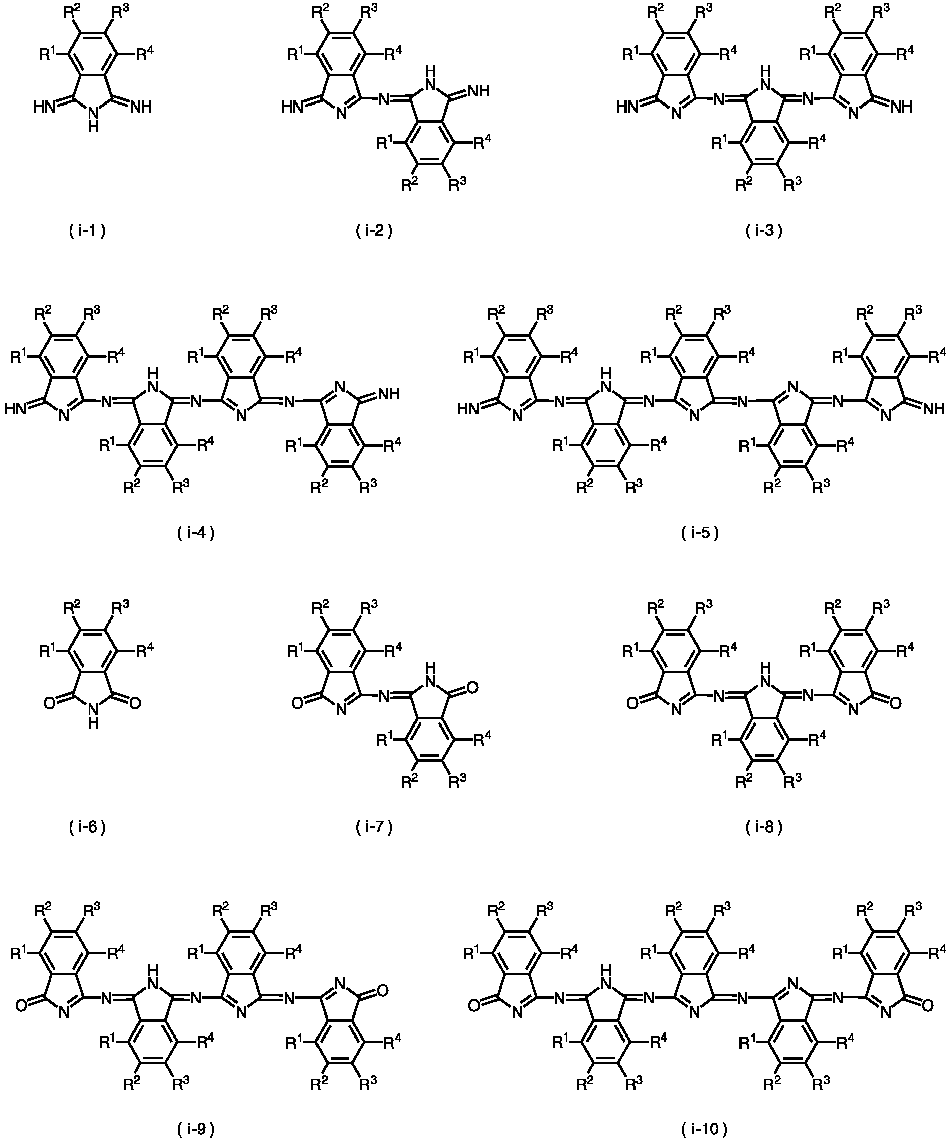 (In the formulae, R¹ to R⁴ each independently represent the same meaning as R¹ to R⁴ in the formula (I).)

### <<Reaction D>>

Compound (II) can be produced by subjecting Compound (I) obtained by Reaction C to a demetallation reaction. The demetallation reaction may be any as long as the reaction suitably proceeds, and a method described in Chemical Communication, 2009, 1970-1971 is exemplified.

The compounds represented by the formulae (IA) to (IIE) can be obtained by producing a phthalocyanine composition by a method described below.

### <Method for Producing Phthalocyanine Composition>

By optimizing the production method described in the section of <Method for producing Compound (I) or (II) > above, a composition that contains one compound or two or more compounds selected from the group of compounds represented by Compounds (I) and (II) and further contains one compound or two or more compounds selected from the group of compounds represented by the formulae (IA) to (IIE) can be produced.

A method for producing a compound represented by the formula (IA), (IIA), (IB), (IIB), (IC), (IIC), (ID), (IID), (IE), or (IIE) will be described below.

From a compound represented by the following formula (DA-2), a compound represented by the following formula (PA-2) is obtained, and from the compound represented by the formula (PA-2), a compound represented by the formula (IA), (IIA), (IB), (IIB), (IC), (IIC), (ID), (IID), (IE), or (IIE) of the invention can be obtained. 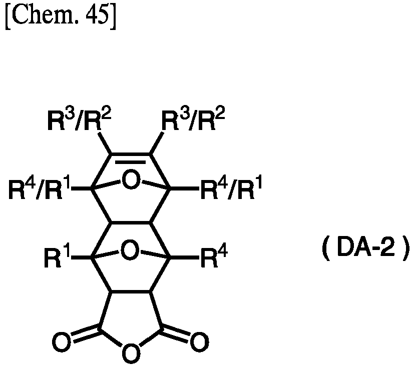 (In the formula, R¹ to R⁴ represent the same meaning as R¹ to R⁴ described above.) 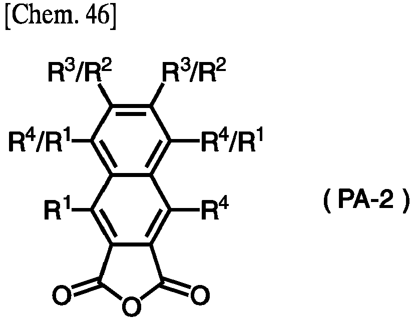 (In the formula, R¹ to R⁴ represent the same meaning as R¹ to R⁴ described above.)

Examples of the compound represented by the formula (DA-2) include, but not limited to, the following Compounds (DA-2-1) to (DA-2-7).

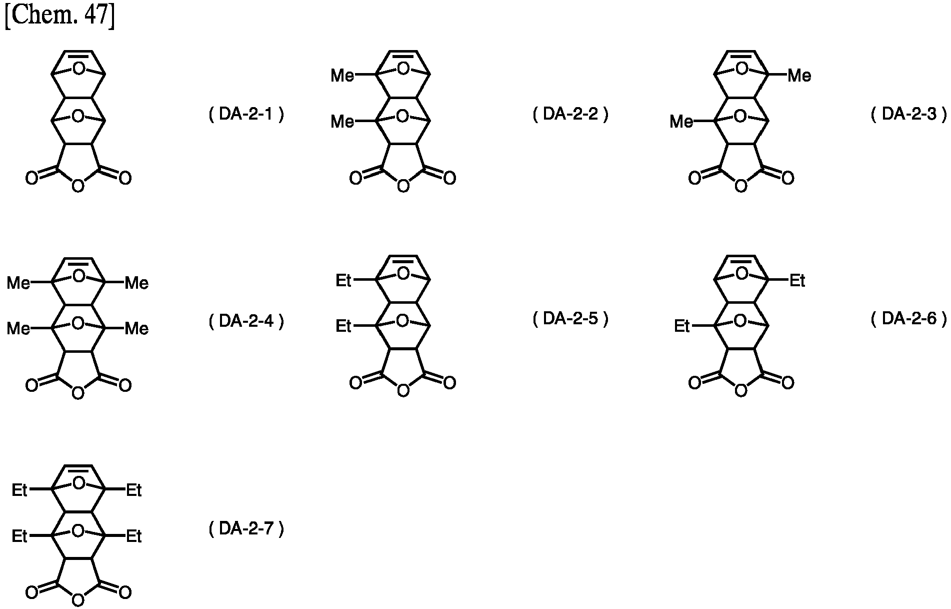

Examples of the compound represented by the formula (PA-2) include, but not limited to, the following Compounds (PA-2-1) to (PA-2-7).

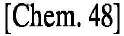

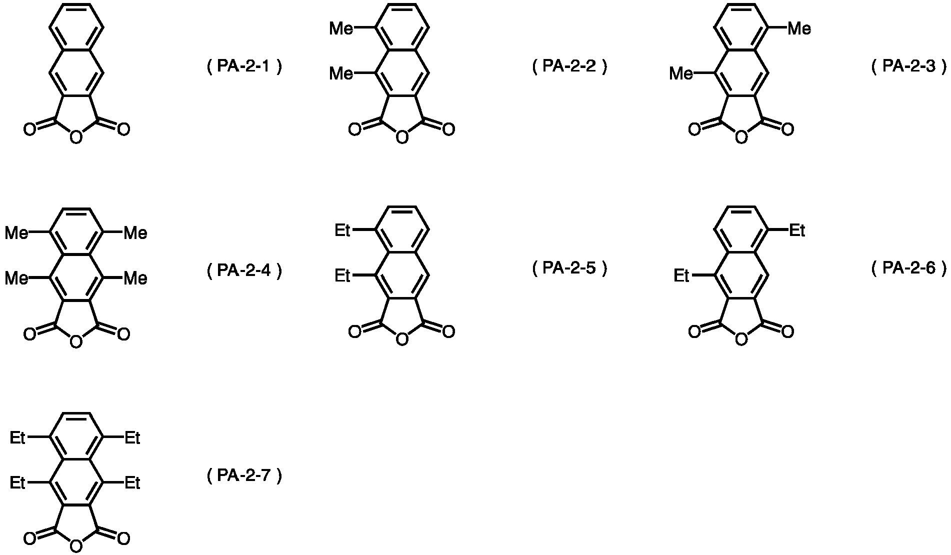

In addition, the compound represented by the formula (DA-2) may be any as long as the reaction suitably proceeds, but is preferably obtained by allowing the compound represented by the formula (FR-1) to react with maleic anhydride. In this reaction, the compound represented by the formula (FR-1) is preferably used in an excess amount by mole relative to maleic anhydride.

When the compound represented by the formula (PA-2) is obtained from the compound represented by the formula (DA-2), a catalyst, which may be any as long as the reaction suitably proceeds, is preferably used.

The catalyst may be any as long as the reaction suitably proceeds, but is preferably hydrochloric acid, sulfuric acid, p-toluenesulfonic acid, methanesulfonic acid, trifluoromethanesulfonic acid, polyphosphoric acid, diphosphoric acid, trifluoroacetic acid, or a compound represented by the following formula (AC). 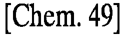 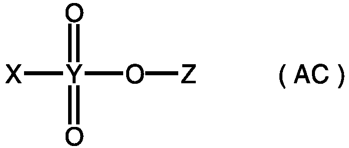 (In the formula, X represents -OH, -ONa, -OK, or -R⁵, R⁵ represents the same meaning as R¹ to R⁴ described above, Y represents a sulfur atom or a phosphorus atom, Z represents a hydrogen atom, -COR⁶, -COH, or -CO-CF₃, and R⁶ represents the same meaning as R¹ to R⁴ described above.)

The amount of the catalyst relative to the compound represented by the formula (DA-2) is preferably 0.1 to 3000% by mole, preferably 0.5 to 2500% by mole, preferably 1 to 2000% by mole, preferably 5 to 1500% by mole, preferably 10 to 1000% by mole, preferably 20 to 500% by mole, preferably 50 to 500% by mole, preferably 70 to 500% by mole, preferably 100 to 500% by mole, preferably 150 to 500% by mole, preferably 200 to 500% by mole, preferably 250 to 500% by mole, preferably 300 to 500% by mole. The lower limit is preferably 0.1% by mole or more, preferably 0.5% by mole or more, preferably 1% by mole or more, preferably 5% by mole or more, preferably 10% by mole, preferably 20% by mole or more, preferably 50% by mole or more, preferably 70% by mole or more, preferably 100% by mole or more, preferably 150% by mole or more, preferably 200% by mole or more, preferably 250% by mole or more, preferably 300% by mole or more. The upper limit is preferably 3000% by mole or less, preferably 2500% by mole or less, preferably 2000% by mole or less, preferably 1500% by mole or less, preferably 1000% by mole or less, preferably 500% by mole or less. The upper limits and the lower limits may be used in any combination.

In this production method, all of R¹ to R⁴ preferably represent a hydrogen atom.

The maleic anhydride is preferably derived from biomass.

The compound represented by the formula (FR-1) is preferably derived from biomass.

It is more preferred that both the maleic anhydride and the compound represented by the formula (FR-1) are derived from biomass.

By the production method, a composition that contains one compound or two or more compounds selected from the group of compounds represented by the formulae (I) and (II) and one compound or two or more compounds selected from the group of compounds represented by the formulae (IA), (IIA), (IB), (IIB), (IC), (IIC), (ID), (IID), (IE), and (IIE) can be obtained.

A specific reaction route of the method for producing the composition which contains one compound or two or more compounds selected from the group of compounds represented by the formulae (I) to (IIE) is shown below. 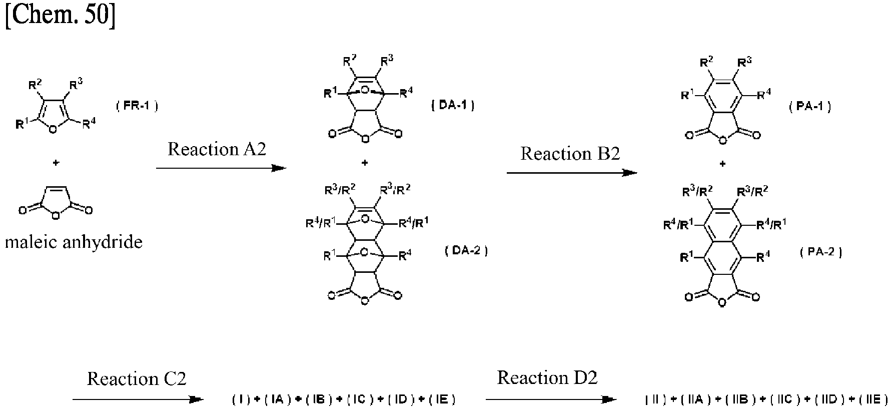 (In the formula, R¹ to R⁴ represent the same meaning as R¹ to R⁴ described above.)

### < <Reaction A2> >

A composition containing Compounds (DA-1) and (DA-2) can be produced by subjecting Compound (FR-1) and maleic anhydride to a Diels-Alder reaction.

The reaction solvent may be any as long as the reaction suitably proceeds, but is preferably chloroform, dioxane, ethyl acetate, an alkylbenzene, toluene, xylene, or diethyl ether.

The reaction temperature may be any as long as the reaction suitably proceeds, but is preferably -10 to 100°C, more preferably 0°C to 80°C, further preferably 10°C to 70°C, and particularly preferably 15°C to 50°C. The lower limit is preferably -10°C or higher, more preferably 0°C or higher, further preferably 10°C or higher, and particularly preferably 15°C or higher. The upper limit is preferably 100°C or lower, more preferably 80°C or lower, further preferably 70°C or lower, and particularly preferably 50°C or lower. For promoting production of Compound (DA-2), the reaction temperature is preferably 0 to 100°C, preferably 10 to 100°C, more preferably 15 to 100°C, further preferably 20 to 100°C, and particularly preferably 25 to 100°C. The lower limit is preferably 0°C or higher, preferably 10°C or higher, more preferably 15°C or higher, further preferably 20°C or higher, and particularly preferably 25°C or higher. For suppressing production of Compound (DA-2), the reaction temperature is preferably -10 to 90°C, preferably -10 to 80°C, more preferably -10 to 70°C, further preferably -10 to 60°C, and particularly preferably -10 to 50°C. The upper limit is preferably 90°C or lower, preferably 80°C or lower, more preferably 70°C or lower, further preferably 60°C or lower, and particularly preferably 50°C or lower.

The reaction pressure may be any as long as the reaction suitably proceeds, but is preferably 0.1 to 5 MPa, more preferably 0.1 to 3 MPa, further preferably 0.1 to 1 MPa, and particularly preferably 0.1 to 0.5 MPa. The lower limit is preferably 0.1 MPa or more, preferably 0.2 MPa or more, preferably 0.3 MPa or more, preferably 0.4 MPa or more. The upper limit is preferably 5 MPa or less, preferably 3 MPa or less, preferably 1 MPa or less, preferably 0.9 MPa or less, preferably 0.8 MPa or less, preferably 0.7 MPa or less, preferably 0.6 MPa or less, preferably 0.5 MPa or less. For promoting production of Compound (DA-2), the reaction pressure is preferably 0.15 to 5 MPa, more preferably 0.2 to 3 MPa, further preferably 0.25 to 1 MPa, and particularly preferably 0.3 to 1 MPa. The lower limit is preferably 0.15 MPa or more, more preferably 0.2 MPa or more, further preferably 0.25 MPa or more, and particularly preferably 0.3 MPa or more. For suppressing production of Compound (DA-2), the reaction pressure is preferably 0.1 to 3 MPa, more preferably 0.1 to 1 MPa, further preferably 0.1 to 0.5 MPa, and particularly preferably 0.1 to 0.4 MPa. The upper limit is preferably 3 MPa or less, more preferably 1 MPa or less, further preferably 0.5 MPa or less, and particularly preferably 0.4 MPa or less.

Further, by varying the equivalents of Compound (FR-1) and maleic anhydride, the ratio of Compound (DA-1) and Compound (DA-2) produced can be controlled. For promoting production of Compound (DA-2), the equivalent of Compound (FR-1) relative to maleic anhydride, which may be any as long as the reaction suitably proceeds, is preferably 2.0 to 15.0, more preferably 4.0 to 14.0, further preferably 6.0 to 13.0, and particularly preferably 8.0 to 12.0. The lower limit is preferably 2.0 or more, more preferably 4.0 or more, further preferably 6.0 or more, and particularly preferably 8.0 or more. The upper limit is preferably 15.0 or less, more preferably 14.0 or less, further preferably 13.0 or less, and particularly preferably 12.0 or less. For suppressing production of Compound (DA-2), the equivalent of Compound (FR-1) relative to maleic anhydride, which may be any as long as the reaction suitably proceeds, is preferably 1.0 to 2.0, more preferably 1.0 to 1.5, further preferably 1.0 to 1.4, and particularly preferably 1.0 to 1.2. The upper limit is preferably 2.0 or less, more preferably 1.5 or less, further preferably 1.4 or less, and particularly preferably 1.2 or less.

### < <Reaction B2> >

By subjecting the composition containing Compounds (DA-1) and (DA-2) obtained by Reaction A2 to a ring-opening dehydration reaction, a composition containing Compounds (PA-1) and (PA-2) can be produced.

The reaction solvent may be any as long as the reaction suitably proceeds, but water, acetonitrile, toluene, xylene, an alkylbenzene or a mixed solvent thereof, or no solvent is preferably used.

The reaction temperature may be any as long as the reaction suitably proceeds, but is preferably 20 to 150°C, more preferably 30 to 120°C, more preferably 40 to 100°C. The lower limit is preferably 20°C or higher, preferably 25°C or higher, preferably 30°C or higher, preferably 35°C or higher, preferably 40°C or higher. The upper limit is preferably 150°C or lower, preferably 140°C or lower, preferably 130°C or lower, preferably 120°C or lower, preferably 110°C or lower, preferably 100°C or lower.

A catalyst is preferably used in the reaction. The catalyst may be any as long as the reaction suitably proceeds, but is preferably hydrochloric acid, sulfuric acid, p-toluenesulfonic acid, methanesulfonic acid, trifluoromethanesulfonic acid, polyphosphoric acid, diphosphoric acid, trifluoroacetic acid, or a compound represented by the following formula (AC). 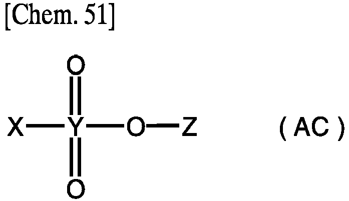 (In the formula, X represents -OH, -ONa, -OK, or -R⁵, R⁵ represents the same meaning as R¹ to R⁴ described above, Y represents a sulfur atom or a phosphorus atom, Z represents a hydrogen atom, -COR⁶, -COH, or -CO-CF₃, and R⁶ represents the same meaning as R¹ to R⁴ described above.)

The amount of the catalyst relative to the total amount of the compounds represented by the formulae (DA-1) and (DA-2) is preferably 0.1 to 3000% by mole, preferably 0.5 to 2500% by mole, preferably 1 to 2000% by mole, preferably 5 to 1500% by mole, preferably 10 to 1000% by mole, preferably 20 to 500% by mole, preferably 50 to 500% by mole, preferably 70 to 500% by mole, preferably 100 to 500% by mole, preferably 150 to 500% by mole, preferably 200 to 500% by mole, preferably 250 to 500% by mole, preferably 300 to 500% by mole. The lower limit is preferably 0.1% by mole or more, preferably 0.5% by mole or more, preferably 1% by mole or more, preferably 5% by mole or more, preferably 10% by mole, preferably 20% by mole or more, preferably 50% by mole or more, preferably 70% by mole or more, preferably 100% by mole or more, preferably 150% by mole or more, preferably 200% by mole or more, preferably 250% by mole or more, preferably 300% by mole or more. The upper limit is preferably 3000% by mole or less, preferably 2500% by mole or less, preferably 2000% by mole or less, preferably 1500% by mole or less, preferably 1000% by mole or less, preferably 500% by mole or less. The upper limits and the lower limits may be used in any combination.

### < <Reaction C2> >

By allowing the composition containing Compounds (PA-1) and (PA-2) obtained by Reaction B2 to react with urea and MX in the presence of a catalyst, a composition that contains one compound or two or more compounds selected from the group of compounds represented by the formula (I) and contains one compound or two or more compounds selected from the group of compounds represented by the formulae (IA), (IB), (IC), (ID), and (IE) can be produced.

The reaction solvent may be any as long as the reaction suitably proceeds, but no solvent or an alkylbenzene is preferably used.

The reaction temperature may be any as long as the reaction suitably proceeds, but is preferably 100 to 250°C, preferably 110 to 240°C, preferably 120 to 230°C, preferably 130 to 220°C, preferably 140 to 210°C, preferably 150 to 200°C. The lower limit is preferably 100°C or higher, preferably 110°C or higher, preferably 120°C or higher, preferably 130°C or higher, preferably 140°C or higher, preferably 150°C or higher. The upper limit is preferably 250°C or lower, preferably 240°C or lower, preferably 230°C or lower, preferably 220°C or lower, preferably 210°C or lower, preferably 200°C or lower.

M in the MX represents a metal atom, and is preferably Al, Si, Sc, Ti, V, Mg, Fe, Co, Ni, Cu, Zn, Ga, Ge, Y, Zr, Nb, In, Sn, or Pb, more preferably Al, Fe, Cu, or Zn, and further preferably Cu or Zn.

X in the MX represents a halogen atom, and is more preferably a chlorine atom.

The catalyst may be any as long as the reaction suitably proceeds, and is preferably a molybdenum catalyst, and more preferably ammonium molybdate tetrahydrate.

By this reaction, compounds represented by the following (i-1) to (i-5) can be produced. 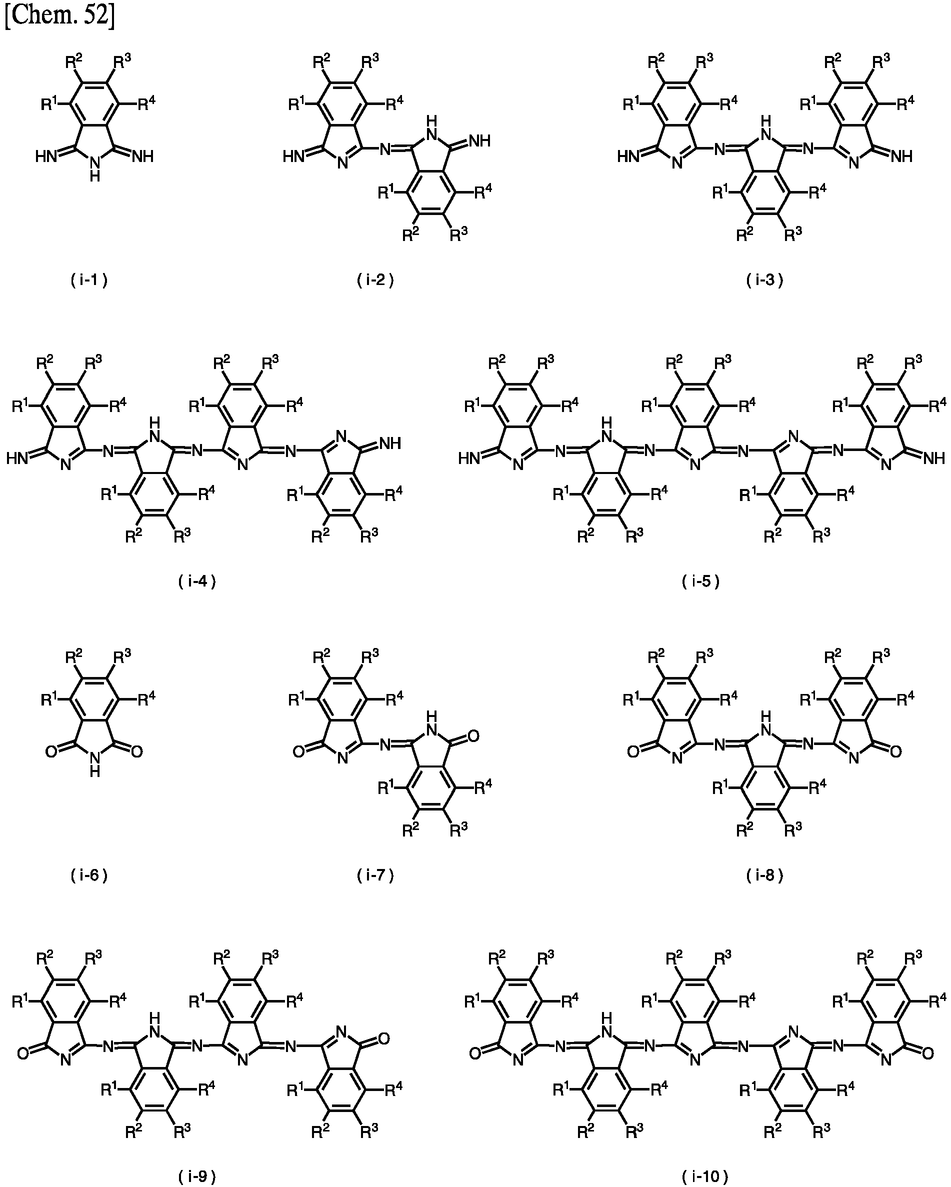 (In the formula, R¹ to R⁴ each independently represent the same meaning as R¹ to R⁴ in the formula (I).)

### < <Reaction D2> >

By subjecting the composition that contains one compound or two or more compounds represented by the formula (I) and contains one compound or two or more compounds selected from the group of compounds represented by the formulae (IA), (IB), (IC), (ID), and (IE) obtained by Reaction C2 to a demetallation reaction, a composition that contains one compound or two or more compounds represented by the formula (II) and one compound or two or more compounds selected from the group of compounds represented by the formulae (IIA), (IIB), (IIC), (IID), and (IIE) can be produced. The demetallation reaction may be any as long as the reaction suitably proceeds, and a method described in Chemical Communication, 2009, 1970-1971 is exemplified.

By such a production as above, a composition that contains one compound or two or more compounds selected from the group of compounds represented by the formulae (I) and (II) and one compound or two or more compounds selected from the group of compounds represented by the formulae (IA) to (IIE) can be obtained.

The content of the compound represented by the formulae (IA) to (IIE) in the composition obtained as above is preferably 0.1 to 40% by weight, preferably 0.1 to 30% by weight, more preferably 0.1 to 20% by weight, further preferably 0.1 to 15% by weight, and particularly preferably 0.1 to 10% by weight. The lower limit is preferably 0.1% by weight or more, preferably 0.5% by weight or more, more preferably 1.0% by weight or more, further preferably 1.5% by weight or more, and particularly preferably 2.0% by weight or more. The upper limit is preferably 40% by weight or less, preferably 30% by weight or less, more preferably 20% by weight or less, further preferably 15% by weight or less, and particularly preferably 10% by weight or less.

The compounds represented by the formulae (I), (II), or (IA) to (IIE) produced by the above method can be further subjected to purification by a known ordinary method to extract only the compound represented by the formula (I) or (II).

The compounds represented by the formulae (I), (II), or (IA) to (IIE) produced by the above method, the compound represented by the formula (I) or (II) extracted by the above purification, and the compounds contained in the composition can be further subjected to halogenation, sulfonation, and imidation by known ordinary methods.

Since the compound of the invention which is represented by the formulae (I) to (IIE) and which contains a radioactive carbon atom ¹⁴C obtained by the above method provides a pigment having a small particle size which is easily further refined as shown in the Examples described later, the dispersibility in a resin in which the pigment is to be dispersed can be enhanced.

In addition, since the compound of the invention can be obtained by the above production method, the numbers and positions of various types of functional groups can be controlled and thus, the hue can be adjusted as desired.

Furthermore, the compound of the invention contains biomass-derived carbon, and thus, contributes to reduction in the environmental load by the carbon neutrality.

The compound of the invention particularly shows a nature as an organic pigment, and the compound can be more suitably used by applying refinement of the pigment particles. Examples of such a treatment include an acid paste method, an acid slurry method, a dry milling method, a solvent method, a salt milling method and the like, and one of the methods can be applied or two or more thereof can be applied in combination.

With the compound of the invention, an additional color material, such as an organic pigment, an organic dye, or an organic pigment derivative, may be used together for the purpose of toning. The material is to be appropriately selected depending on the application, and the compound of the invention may be used alone or two or more of the compounds of the invention may be used in combination according to the application.

Any of known pigment dyes and the like may be used as a color material usable together.

### (Application of Compound of Present Invention)

The compound of the invention can be applied for various applications. For example, the compound can be used as a pigment composition, and, with another resin, rubber, additive, pigment, dye or the like mixed as needed, may be adjusted for and used in a coating material or printing marker on a cosmetic, a medicine, or a pesticide, a stationery product, a writing tool, a printing ink, an inkjet ink, a metal ink, a paint, a plastic colorant, a color toner, a color filter, an organic semiconductor material, or a near infrared absorbent for laser welding which utilizes the strong absorption of near infrared light. Some examples of the above applications will be shown below.

### < Cosmetic Application >

The compound of the invention can be used as a cosmetic. The cosmetic used is not particularly limited, and the compound of the invention can be used in various types of cosmetics.

The cosmetic may be any type of cosmetic as long as a function can be effectively exhibited. The cosmetic may be a lotion, a cream gel, a spray, or the like. Examples of the cosmetic include skin care cosmetics, such as a face washing agent, a makeup remover, a face lotion, an essence, a facial pack, a protective milky lotion, a protective cream, a whitening cosmetic, and an ultraviolet blocking cosmetic, makeup cosmetics, such as a foundation, a face powder, a makeup base, a lipstick, an eye makeup, a cheek rouge, and a nail enamel, hair care cosmetics, such as a shampoo, a hair rinse, a hair treatment, a hair dressing, a permanent waving agent, a hair dye, and a hair growth agent, body care cosmetics, such as a body washing cosmetic, a deodorant cosmetic, and a bathing agent.

The amount of the compound of the invention used in the cosmetic can be appropriately set depending on the type of the cosmetic. The content in the cosmetic is usually in the range of 0.1 to 99% by mass, and is generally preferably in the range of 0.1 to 10% by mass. On the other hand, in a makeup cosmetic that has a purpose of coloring, the content is preferably in the range of 5 to 80% by mass, further preferably in the range of 10 to 70% by mass, and most preferably in the range of 20 to 60% by mass. When the amount of the compound of the invention contained in the cosmetic is in the above ranges, a function, such as coloring, can be effectively exhibited and functions required for a cosmetic can also be maintained.

Depending on the type of the cosmetic, the cosmetic can contain, in addition to the compound of the invention, a carrier, a pigment, an oil, a sterol, an amino acid, a moisturizer, a powder, a colorant, a pH modifier, a perfume, an essential oil, a cosmetic active component, a vitamin, an essential fatty acid, a sphingolipid, a self-tanning agent, an excipient, a filler, an emulsifier, an antioxidant, a surfactant, a chelating agent, a gelling agent, a concentrating agent, an emollient, a humectant, a moisturizer, a mineral, a viscosity modifier, a fluidity modifier, a keratolytic agent, a retinoid, a hormone compound, an *α-*hydroxy acid, an *α* -keto acid, an antimycobacterial agent, an antifungal agent, an antibacterial agent, an antiviral agent, an analgesic, an antiallergic agent, an antihistamine agent, an anti-inflammatory agent, an anti-irritant agent, an antitumor agent, an immune system booster, an immune system suppressor, an anti-acne agent, an anesthetic, an antiseptic, an insecticide, a skin cooling compound, a skin protective agent, a skin penetration enhancer, an exfoliant, a lubricant, a fragrance, a dyeing agent, a decolorant, a hypopigmenting agent, a preservative, a stabilizer, a medicine, a photostabilizer, and a spherical powder, which are acceptable as a cosmetic component.

The cosmetic can be produced by mixing the compound of the invention and other cosmetic components.

The cosmetic containing the compound of the invention can be used in the same manner as ordinary cosmetics according to the type of the cosmetic.

### < Printing Ink Application >

The compound of the invention can be used for producing a low-viscosity ink superior in fluidity, and is suitable for a pigment for a photogravure printing ink or a flexographic printing ink.

An ink is constituted of a binder resin, a solvent, a pigment, and various additives.

Examples of the binder resin include a nitrocellulose resin, a polyamide resin, a polyurethane resin, and an acrylic resin.

Examples of the solvent include aromatic organic solvents, such as toluene and xylene, ketone solvents, such as methyl ethyl ketone, methyl isobutyl ketone, cyclohexanone, 2-heptanone, and 3-heptanone, ester solvents, such as ethyl acetate, n-propyl acetate, isopropyl acetate, and isobutyl acetate, alcohol solvents, such as methanol, ethanol, n-propanol, isopropanol, n-butanol, isobutanol, and t-butanol, and glycol ether solvents, such as ethylene glycol monomethyl ether, ethylene glycol monoethyl ether, ethylene glycol mono-n-propyl ether, ethylene glycol mono-i-propyl ether, ethylene glycol mono-n-butyl ether, ethylene glycol mono-i-butyl ether, ethylene glycol mono-t-butyl ether, propylene glycol monomethyl ether, propylene glycol monoethyl ether, propylene glycol mono-n-propyl ether, propylene glycol mono-i-propyl ether, propylene glycol mono-n-butyl ether, propylene glycol mono-i-butyl ether, propylene glycol mono-t-butyl ether, ethylene glycol dimethyl ether, ethylene glycol diethyl ether, ethylene glycol di-n-propyl ether, ethylene glycol di-i-propyl ether, ethylene glycol di-n-butyl ether, ethylene glycol di-i-butyl ether, ethylene glycol di-t-butyl ether, propylene glycol dimethyl ether, propylene glycol diethyl ether, propylene glycol di-n-propyl ether, propylene glycol di-i-propyl ether, propylene glycol di-n-butyl ether, propylene glycol di-i-butyl ether, propylene glycol di-t-butyl ether, diethylene glycol monomethyl ether, diethylene glycol monoethyl ether, diethylene glycol mono-n-propyl ether, diethylene glycol mono-i-propyl ether, diethylene glycol mono-n-butyl ether, diethylene glycol mono-i-butyl ether, diethylene glycol mono-t-butyl ether, diethylene glycol dimethyl ether, diethylene glycol diethyl ether, diethylene glycol di-n-propyl ether, diethylene glycol di-i-propyl ether, diethylene glycol di-n-butyl ether, diethylene glycol di-i-butyl ether, diethylene glycol di-t-butyl ether, dipropylene glycol monomethyl ether, dipropylene glycol monoethyl ether, dipropylene glycol mono-n-propyl ether, dipropylene glycol mono-i-propyl ether, dipropylene glycol mono-n-butyl ether, dipropylene glycol mono-i-butyl ether, dipropylene glycol mono-t-butyl ether, dipropylene glycol dimethyl ether, dipropylene glycol diethyl ether, dipropylene glycol di-n-propyl ether, dipropylene glycol di-i-propyl ether, dipropylene glycol di-n-butyl ether, dipropylene glycol di-i-butyl ether, dipropylene glycol di-t-butyl ether, ethylene glycol monomethyl ether acetate, ethylene glycol monoethyl ether acetate, propylene glycol monoethyl ether acetate, propylene glycol monomethyl ether acetate, diethylene glycol monomethyl ether acetate, and diethylene glycol monoethyl ether acetate.

Examples of the additives include surfactants, such as an anionic surfactant, a nonionic surfactant, a cationic surfactant, and an amphoteric surfactant, rosins, such as a gum rosin, a polymerized rosin, a disproportionated rosin, a hydrogenated rosin, a maleated rosin, a hardened rosin, and a phthalic alkyd resin, a pigment derivative, a dispersant, a humectant, an adhesive assistant, a leveling agent, an antifoaming agent, an antistatic agent, a trapping agent, an antiblocking agent, and a wax component.

The printing ink prepared as above can be used for printing on a paper, a synthetic paper, a thermoplastic resin film, a plastic product, a steel plate, or the like, and is useful as an ink for photogravure printing using a gravure printing plate of an electro-engraving intaglio plate or the like, an ink for flexographic printing using a flexographic printing plate of a resin plate or the like, for example. The printing ink is once allowed to adhere on and transferred to a printing plate or printing pattern, and then, only the ink is again allowed to adhere on the substrate, and is dried as needed, whereby a printed article is formed. The printed article can also be used as a component of a laminate with another substrate or the like.

### <Paint Application>

The compound of the invention can be incorporated into a paint as a colorant.

Examples of the resin used in a paint are various, and include an acrylic resin, a melamine resin, an epoxy resin, a polyester resin, a polyurethane resin, a polyamide resin, and a phenol resin.

Examples of the solvent used in the paint include aromatic solvents, such as toluene, xylene, and methoxybenzene, acetate ester solvents, such as ethyl acetate, butyl acetate, propylene glycol monomethyl ether acetate, and propylene glycol monoethyl ether acetate, propionate solvents, such as ethoxyethyl propionate, alcohol solvents, such as methanol, ethanol, propanol, n-butanol, and isobutanol, ether solvents, such as butylcellosolve, propylene glycol monomethyl ether, diethylene glycol ethyl ether, and diethylene glycol dimethyl ether, ketone solvents, such as methyl ethyl ketone, methyl isobutyl ketone, and cyclohexanone, aliphatic hydrocarbon solvents, such as hexane, nitrogen compound solvents, such as N,N-dimethylformamide, *γ* -butyrolactam, N-methyl-2-pyrrolidone, aniline, and pyridine, lactone solvents, such as *γ* -butyrolactone, carbamate ester, such as a mixture of methyl carbamate and ethyl carbamate at 48:52, and water. As the solvent, a propionate, an alcohol, an ether, a ketone, a nitrogen compound, and a lactone solvent, and a water-soluble polar solvent, such as water, are particularly suitable.

When a pigment additive and/or a pigment composition is dispersed in and mixed with a liquid resin to form a resin composition for a paint, general additives, for example, dispersants, fillers, paint assistants, drying agents, plasticizers, and/or auxiliary pigments can be used. This is achieved by gathering one each of or two or more each which are mixed together of all kinds of the components, or adding all the components at once, followed by dispersion or mixing.

Examples of a disperser for dispersing the composition containing the compound of the invention prepared according to the application as described above include, but not limited to, known dispersers, such as a disper, a homomixer, a paint conditioner, Scandex, a bead mill, Atritor, a boll mill, a two roll, a three roll, and a pressure kneader. The dispersion of the pigment composition is performed with a resin and a solvent added so as to provide a viscosity at which dispersion is possible with the disperser. A high concentration paint base after dispersion has a solid content of 5 to 20%, and the resin and the solvent are further mixed therewith to provide a paint, which is then subjected to use.

A coating film is formed with the thus prepared paint, whereby a painted article can be produced.

<Inkjet Ink Application>

The compound of the invention can be suitably used in an inkjet ink, and in particular, can be suitably used in an aqueous inkjet ink in the form of an aqueous pigment dispersion in which the compound is dispersed by using a pigment dispersant or the like. The aqueous pigment dispersion can be prepared by producing a high concentration aqueous dispersion (pigment paste) of the fused polycyclic organic pigment of the invention, diluting the high concentration aqueous dispersion with a water-soluble solvent and/or water, and adding other additives as required.

The method for dispersing the compound of the invention in the water-soluble solvent and/or water to obtain a pigment past is not particularly limited, and a known dispersion method is preferably used. Regarding a dispersant used herein, a known pigment dispersant may be used for dispersion in water, or a surfactant may be used. The pigment dispersant is preferably an aqueous resin, and preferred examples thereof include a polyvinyl alcohol, a polyvinyl pyrrolidone, a urethane resin having an anionic group or a cationic group, and a radical copolymer resin having an anionic group or a cationic group. Examples of the radical copolymer resin having an anionic group or a cationic group include acrylic resins, such as an acrylic acid-acrylate ester copolymer, styrene-acrylic resins, such as a styrene-acrylic acid copolymer, a styrene-methacrylic acid copolymer, a styrene-methacrylic acid-acrylate ester copolymer, a styrene- *α* -methylstyrene-acrylic acid copolymer, and a styrene-*α* -methylstyrene-acrylic acid -acrylate ester copolymer, a styrene-maleic acid copolymer, a styrene-maleic anhydride copolymer, and a vinylnaphthalene-acrylic acid copolymer, and salts of the aqueous resins.

Examples of compounds for forming the salts of the copolymers include alkali metal hydroxides, such as sodium hydroxide, potassium hydroxide, and lithium hydroxide, and diethylamine, ammonia, ethylamine, triethylamine, propylamine, isopropylamine, dipropylamine, butylamine, isobutylamine, triethanolamine, diethanolamine, aminomethylpropanol, and morpholine. The amount of the compound used for forming the salts is preferably the neutralization equivalent of the copolymer or more.

Of course, a commercial product can also be used. Examples of the commercial product include AJISPER PB series which are products of Ajinomoto Fine-Techno Co., Inc., DISPERBYK series and BYK series manufactured by BYK JAPAN KK, and EFKA series manufactured by BASF Japan Ltd.

As a dispersion method, for example, the following (1) to (3) can be shown.
(1) A method in which a pigment is added to an aqueous medium containing a pigment dispersant and water, and then, the pigment is dispersed in the aqueous medium with a stirring-dispersing apparatus to thereby prepare a pigment paste.
(2) A method in which a pigment and a pigment dispersant are kneaded with a kneader, such as a two roll or a mixer, the resulting kneaded product is added into an aqueous medium containing water, and a pigment paste is prepared with a stirring-dispersing apparatus.
(3) A method in which a pigment dispersant is dissolved in an organic solvent which has a compatibility with water, such as methyl ethyl ketone or tetrahydrofuran, a pigment is added to the resulting solution, the pigment is then dispersed in the organic solution with a stirring-dispersing apparatus, phase-transfer emulsification is then performed with an aqueous medium, and the organic solvent is removed by distillation, thereby preparing a pigment paste.

The kneader is not particularly limited, and examples thereof include a Henschel mixer, a pressure kneader, a Bunbury mixer, and a planetary mixer. Furthermore, the stirring-dispersing apparatus is not particularly limited, and examples thereof include an ultrasonic homogenizer, a high-pressure homogenizer, a paint shaker, a ball mill, a roll mill, a sand mill, a sand grinder, DYNO-MILL, DISPERMAT, an SC mill, and Nanomizer. One of the kneaders may be used alone or two or more thereof may be used in combination.

The amount of the fused polycyclic organic pigment in the pigment paste is preferably 5 to 60% by mass, and more preferably 10 to 50% by mass. When the amount of the pigment is less than 5% by mass, the color of an aqueous ink prepared from the pigment paste tends to be not enough to achieve a sufficient image concentration. On the other hand, when the amount is more than 60% by mass, the dispersion stability of the pigment in the pigment paste tends to decrease.

In addition, since coarse particles cause nozzle clogging or otherwise cause deterioration of image characteristics, coarse particles are preferably removed by centrifugation, filtration, or the like before or after preparing the ink.

After the dispersion step, an impurity removal step by an ion exchange treatment or an ultrafiltration treatment may be performed, followed by a post treatment. By the ion exchange treatment, a cation, an anion, and other ionic substances (divalent metal ion, etc.) can be removed, and by the ultrafiltration treatment, impurity-dissolving substances (residual substances in synthesis of the pigment, excess components in the dispersion composition, a resin that is not adsorbed on the organic pigment, contaminating substances, and the like) can be removed. In the ion exchange treatment, a known ion exchange resin is used. In the ultrafiltration treatment, a known ultrafiltration membrane is used, which may be a general type or a double capability type.

After preparing the pigment paste, the pigment paste is appropriately diluted and an additive is added as required, whereby an aqueous pigment dispersion according to the purpose is provided. When the aqueous pigment dispersion is applied to an inkjet recording ink, a water-soluble solvent and/or water, an anionic group-containing organic polymer compound as a binder, and the like are further added, a humectant (dry suppressor), a penetrant, or other additive is added as required for a desired physical property, thus preparing an ink. After preparing the ink, a centrifugation step or filtration step may be additionally performed.

The physical characteristics of the ink are not particularly limited, but, in view of ejectability for an inkjet ink, the viscosity is preferably 1 to 10 (mPa·s), the surface tension is preferably 20 to 50 (mN/m), and the pigment concentration is preferably 1 to 10% by mass.

The humectant is added for the purpose of preventing drying of the ink. The content of the humectant in the ink for the purpose of preventing drying is preferably 3 to 50% by mass. The humectant used in the invention is not particularly limited, but is preferably one that is miscible with water to have an effect of preventing clogging of an inkjet printer head. Examples thereof include glycerol, ethylene glycol, diethylene glycol, triethylene glycol, polyethylene glycol having a molecular weight of 2000 or less, propylene glycol, dipropylene glycol, tripropylene glycol, 1,3-propylene glycol, isopropylene glycol, isobutylene glycol, 1,4-butanediol, 1,3-butanediol, 1,5-pentanediol, 1,6-hexanediol, meso-erythritol, and pentaerythritol. Among them, propylene glycol or 1,3-butylglycol contained provides effects of giving stability and superior ink drying ability and ejection performance.

The penetrant is added for the purpose of improving penetration into a medium to be recorded and adjusting the dot diameter on a recording medium. Examples of the penetrant include a lower alcohol, such as ethanol or isopropyl alcohol, alkyl alcohol ethylene oxide adducts, such as ethylene glycol hexyl ether or diethylene glycol butyl ether, and alkyl alcohol propylene oxide adducts, such as propylene glycol propyl ether.

The surfactant is added for adjusting the ink characteristics, such as surface tension. The surfactant that can be added for this purpose is not particularly limited, and examples thereof include various anionic surfactants, nonionic surfactants, cationic surfactants, and amphoteric surfactants. Among them, anionic surfactants or nonionic surfactants are preferred.

Examples of the anionic surfactant include an alkylbenzene sulfonic acid salt, an alkylphenyl sulfonic acid salt, an alkylnaphthalene sulfonic acid salt, a higher fatty acid salt, a higher fatty acid ester sulfuric acid ester salt, a higher fatty acid ester sulfonic acid salt, a higher alcohol ether sulfuric acid ester salt and sulfonic acid salt, a higher alkyl sulfosuccinic acid salt, a polyoxyethylene alkyl ether carboxylic acid salt, a polyoxyethylene alkyl ether sulfuric acid salt, an alkylphosphoric acid salt, and a polyoxyethylene alkyl ether phosphoric acid salt. Specific examples thereof include a dodecylbenzenesulfonic acid salt, an isopropylnaphthalenesulfonic acid salt, a monobutylphenylphenol monosulfonic acid salt, a monobutylbiphenylsulfonic acid salt, and a dibutylphenylphenol disulfonic acid salt.

Examples of the nonionic surfactant include a polyoxyethylene alkyl ether, a polyoxyethylene alkylphenyl ether, a polyoxyethylene fatty acid ester, a sorbitan fatty acid ester, a polyoxyethylene sorbitan fatty acid ester, a polyoxyethylene sorbitol fatty acid ester, a glycerol fatty acid ester, a polyoxyethylene glycerol fatty acid ester, a polyglycerol fatty acid ester, a sucrose fatty acid ester, a polyoxyethylene alkyl amine, a polyoxyethylene fatty acid amide, a fatty acid alkylol amide, an alkyl alkanol amide, acetylene glycol, an acetylene glycol oxyethylene adduct, and a polyethylene glycol polypropylene glycol block copolymer. Among them, polyoxyethylene nonylphenyl ether, polyoxyethylene octylphenyl ether, polyoxyethylene dodecylphenyl ether, a polyoxyethylene alkyl ether, a polyoxyethylene fatty acid ester, a sorbitan fatty acid ester, a polyoxyethylene sorbitan fatty acid ester, a fatty acid alkylol amide, acetylene glycol, an acetylene glycol oxyethylene adduct, and a polyethylene glycol polypropylene glycol block copolymer are preferred.

Other examples of the surfactant that can be used include a silicone surfactant, such as a polysiloxane oxyethylene adduct; fluorosurfactants, such as a perfluoroalkyl carboxylic acid salt, a perfluoroalkyl sulfonic acid salt, and an oxyethylene perfluoroalkyl ether; and bio surfactants, such as spiculisporic acid, rhamnolipid, and lysolecithin.

The surfactants can be used alone or two or more thereof can be used in mixture. When a surfactant is added, the amount of the surfactant added based on the total mass of the ink is preferably in the range of 0.001 to 2% by mass, more preferably 0.001 to 1.5% by mass, and further preferably in the range of 0.01 to 1% by mass. With an amount of the surfactant added of less than 0.001% by mass, the effect of addition of the surfactant tends to be not achieved, whereas with an amount of more than 2% by mass, a problem of blotting of the image and the like are liable to occur.

In addition, an antiseptic, a viscosity modifier, a pH modifier, a chelating agent, a plasticizer, an antioxidant, an ultraviolet ray absorber, or the like can be added as required.

### < Plastic Application >

The compound of the invention can be used for coloring a plastic. When a colored plastic molded article is to be obtained, a thermoplastic resin (plastic) for thermoforming, such as injection molding or press molding, for example, a polyolefin, such as polyethylene or polypropylene, or a polyvinyl chloride resin, is used. The compound of the invention can be used by being kneaded into such a resin by a conventionally known method.

### < Toner Application >

The compound of the invention can be used for coloring a toner.

When an electrostatic charge image developing toner is to be obtained, for example, a thermoplastic resin that is liable to form a solid film at normal temperature, such as a polyester resin, a polyamide resin, a styrene resin, or an acrylic resin, is used as a dispersing resin.

The electrostatic charge image developing toner produced by using the compound of the invention as a component can be used as a one-component color magnetic toner which contains a magnetic substance in the toner (magnetic one-component image developing color toner), a nonmagnetic one-component color color toner which contains no magnetic substance (nonmagnetic one-component image developing color toner), or a two-component color image developing color toner in which a carrier is mixed (two-component image developing color toner).

The one-component color magnetic toner can be constituted of, for example, a colorant, a binder resin, a magnetic powder, other additives, such as a charge control agent (CCA) and a mold release agent, and the like, as in a generally used one.

The amount of the compound of the invention used based on the electrostatic charge image developing toner is not particularly limited, but is preferably 0.5 to 25 parts by mass relative to 100 parts by mass of a binder resin, and from the viewpoint of making the electrification performance of the colorant itself further significant, is further preferably 4 to 10 parts by mass relative to 100 parts by mass of the binder resin.

As the binder resin used in the electrostatic charge image developing toner, any of the known conventional resins which are exemplified as the above-mentioned thermoplastic resin can be used, and any of synthetic resins, natural resins, natural rubbers, synthetic rubbers, synthetic waxes and the like that show adhesiveness under heat or pressure can be used.

### < Color Filter Application >

The compound of the invention can be used for forming a pattern of a green pixel part of a color filter by a known method. Typically, a coloring composition for a color filter containing the compound of the invention and a photosensitive resin as essential components (more specifically, a photosensitive composition for a color filter green pixel part) can be obtained.

For preparing the photosensitive composition for a color filter green pixel part, for example, the compound of the invention, a photosensitive resin, a photopolymerization initiator, and an organic solvent that dissolves the resin are mixed as essential components. As a production method thereof, a method in which the compound of the invention and an organic solvent, and a dispersant as required, are used to prepare a dispersion, and a photosensitive resin or the like is added thereto is generally used.

In the compound of the invention used in the photosensitive composition for a color filter green pixel part, a yellow pigment can be used, as required.

Examples of the dispersant which is used as required include DISPERBYK (registered tradename) 130, 161, 162, 163 and 170, DISPERBYK LPN-6919 and DISPERBYK LPN-21116, and the like from BYK JAPAN KK. In addition, a leveling agent, a coupling agent, a cationic surfactant, or the like can be used together.

Examples of the organic solvent include aromatic solvents, such as toluene, xylene, and methoxybenzene, acetate ester solvents, such as ethyl acetate, butyl acetate, propylene glycol monomethyl ether acetate, and propylene glycol monoethyl ether acetate, propionate solvents, such as ethoxyethyl propionate, alcohol solvents, such as methanol and ethanol, ether solvents, such as butylcellosolve, propylene glycol monomethyl ether, diethylene glycol ethyl ether, and diethylene glycol dimethyl ether, ketone solvents, such as methyl ethyl ketone, methyl isobutyl ketone, and cyclohexanone, aliphatic hydrocarbon solvents, such as hexane, nitrogen compound solvents, such as N,N-dimethylformamide, *γ*-butyrolactam, N-methyl-2-pyrrolidone, aniline, and pyridine, lactone solvents, such as *γ*-butyrolactone, carbamate ester, such as a mixture of methyl carbamate and ethyl carbamate at 48:52, and water. As the organic solvent, a water-soluble polar solvent, such as a propionate, an alcohol, an ether, a ketone, a nitrogen compound, a lactone solvent, or water is particularly suitable.

Examples of the photosensitive resin that can be used include thermoplastic resins, such as a urethane resin, an acrylic resin, a polyamide acid resin, a polyimide resin, a styrene maleic acid resin, and a styrene maleic anhydride resin, and photopolymerizable monomers, for example, bifunctional monomers, such as 1,6-hexanediol diacrylate, ethylene glycol diacrylate, neopentyl glycol diacrylate, triethylene glycol diacrylate, bis(acryloxyethoxy) bisphenol A, and 3-methylpentanediol diacrylate, and polyfunctional monomers, such as trimethylolpropane triacrylate, pentaerythritol triacrylate, tris(2-hydroxyethyl) isocyanate, dipentaerythritol hexaacrylate, and dipentaerythritolpentaacrylate.
Examples of the photopolymerization initiator include acetophenone, benzophenone, benzyl dimethyl ketal, benzoyl peroxide, 2-chlorothioxanthone, 1,3-bis(4'-azidobenzal)-2-propane, 1,3-bis(4'-azidobenzal)-2-propane-2'-sulfonic acid, and 4,4'-diazidostilbene-2,2'-disulfonic acid.

The thus prepared photosensitive composition for a color filter green pixel part is formed into a color filter by subjecting the photosensitive composition to pattern exposure to an ultraviolet ray via a photomask, and then, washing the non-exposed part with an organic solvent, an alkali water, or the like.

### Examples

Hereinbelow, the invention is further described in detail with reference to Examples, but the invention is not to be limited to the Examples. The "%" in the compositions of Examples means "% by mass".

### (Synthetic Example 1) Synthesis 1 of Mixture of Compound (DA-1-1) and Compound (DA-2-1) by Diels-Alder Reaction of Furan and Maleic Anhydride

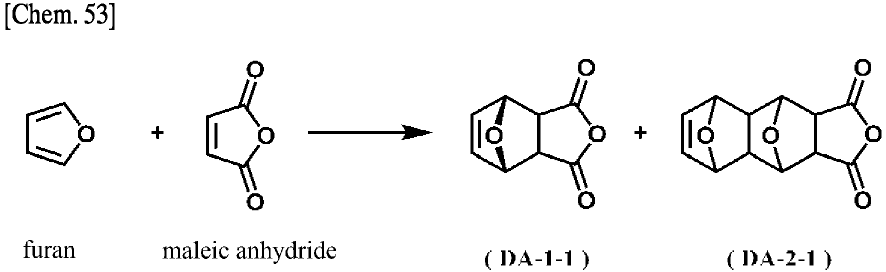

Under a nitrogen atmosphere, in a reactor equipped with a stirrer, a biomass-derived maleic anhydride (24.01 g) was dissolved in diethyl ether (250 mL), a biomass-derived furan (25.00 g) was put therein, and the mixture was reacted at room temperature and 0.25 MPa for 18 hours. Subsequently, a white crude product was separated through a filter, was washed with diethyl ether, and then, was dried under vacuum to obtain a mixture (31.70 g) of Compound (DA-1-1) and Compound (DA-2-1). The ratio of Compound (DA-1-1) and Compound (DA-2-1) was 97% and 0.94%.

### (Example 1) Synthesis of Phthalocyanine Compound (Pc-1) form Mixture of Compound (DA-1-1) and Compound (DA-2-1)

Into a reactor equipped with a stirrer, a thermometer, a dropping funnel, and a cooling pipe, acetic anhydride (21.0 g) and methanesulfonic acid (148.0 g) were added, and the mixture was cooled with ice with stirring. To the reaction mixture, the mixture of Compound (DA-1-1) and Compound (DA-2-1) (10.0 g) obtained in Synthetic Example 1 was slowly added dropwise. After stirring at room temperature for 2 hours, the temperature was increased to 80°C, followed by further stirring for 4 hours. Subsequently, the reaction mixture was cooled to room temperature and toluene (50 mL) was added thereto. The organic layer was separated, and the aqueous layer was extracted again with toluene (50 mL × 2). The obtained organic layers were combined and then washed with water, a saturated saline solution, and an aqueous sodium hydrogen carbonate solution in this order. The solution was concentrated to obtain a white solid of a mixture of phthalic anhydride and 2,3-naphthalenedicarboxylic anhydride.

Subsequently, into a reactor equipped with a stirrer, a thermometer, a dropping funnel, and a cooling pipe, the obtained white solid, urea (9.7 g), copper chloride (1.25 g), ammonium molybdate tetrahydrate (0.03 g), and alkylbenzene (20 mL) were added. The mixture was heated to 190°C with stirring. After stirring for 2 hours, the reaction mixture was cooled to room temperature, and the crude product was separated through a filter and was washed with water and acid in this order, followed by washing until a neutral solution was obtained. The product was dried overnight in an oven to obtain Pc-1 (8.5 g). The Pc-1 had a pMC of 100%, and contained a radioactive carbon atom ¹⁴C.

### (Synthetic Example 2) Synthesis 2 of Mixture of Compound (DA-1-1) and Compound (DA-2-1) by Diels-Alder Reaction of Furan and Maleic Anhydride

Under a nitrogen atmosphere, into a reactor equipped with a stirrer, a petroleum-derived maleic anhydride (24.01 g) was dissolved in diethyl ether (250mL), a biomass-derived furan (25.00 g) was put therein, and the mixture was reacted at room temperature and 0.25 MPa for 18 hours. Subsequently, the white crude product was separated through a filter, was washed with diethyl ether, and was dried under vacuum to obtain a mixture of Compound (DA-1-1) and Compound (DA-2-1) (32.54 g). The ratio of Compound (DA-1-1) and Compound (DA-2-1) was 97% and 0.86%.

### (Example 2) Synthesis of Phthalocyanine Compound (Pc-2) from Mixture of Compound (DA-1-1) and Compound (DA-2-1)

Into a reactor equipped with a stirrer, a thermometer, a dropping funnel, and a cooling pipe, acetic anhydride (13.8 g) and methanesulfonic acid (73.9 g) were added, and the mixture was cooled with ice with stirring. To the reaction mixture, the mixture of Compound (DA-1-1) and Compound (DA-2-1) (5.0 g) obtained in Synthetic Example 2 was slowly added dropwise. After stirring at room temperature for 2 hours, the temperature was increased to 80°C, followed by further stirring for 4 hours. Subsequently, the reaction mixture was cooled to room temperature and toluene (30 mL) was added thereto. The organic layer was separated, and the aqueous layer was extracted again with toluene (30 mL × 2). The obtained organic layers were combined and then washed with water, a saturated saline solution, and an aqueous sodium hydrogen carbonate solution in this order. The solution was concentrated to obtain a white solid of a mixture of phthalic anhydride and 2,3-naphthalene dicarboxylic anhydride.

Subsequently, into a reactor equipped with a stirrer, a thermometer, a dropping funnel, and a cooling pipe, the obtained white solid, urea (5.3 g), copper chloride (0.68 g), ammonium molybdate tetrahydrate (0.02 g), and an alkylbenzene (10 mL) were added, and were heated to 190°C with stirring. After stirring for 2 hours, the reaction mixture was cooled to room temperature, and the crude product was separated through a filter, and was washed with water and acid in this order, followed by washing until a neutral solution was obtained. The product was dried overnight in an oven to obtain Pc-2 (3.6 g). The Pc-2 had a pMC of 50% and contained a radioactive carbon atom ¹⁴C.

### (Comparative Example 1) Synthesis of Comparative Phthalocyanine (RPc-1)

Comparative Phthalocyanine RPc-1 (8.75 g) was produced according to the method described in Chem. Commun., 2009, 1970-1971 except for using an alkylbenzene in place of nitrobenzene. The RPc-1 had a pMC of 0% and contained no radioactive carbon atom ¹⁴C.

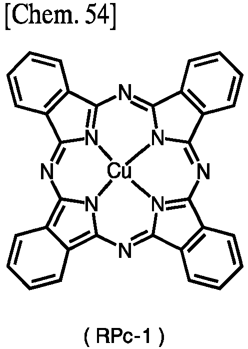

### (Mass Analysis)

The phthalocyanine composition obtained in Example 1 and the like were subjected to mass analysis. As the analytical method, 5 mg of the obtained composition or the like was dissolved in THF and was subjected to FD-MS JMS-T100GC (manufactured by JEOL). The results are shown in the following table.

**[Table 1]**

| | m/z (m/z) | | | | | | | | | | | Total |
|---|---|---|---|---|---|---|---|---|---|---|---|---|
| Composition | 128 | 478 | 520 | 564 | 568 | 575 | 591 | 609 | 611 | 625 | 734 | |
| Pc-1 | 0.2 | 0.1 | 0.1 | 0.5 | 1.1 | 94.6 | 0.1 | 0.3 | - | 2.8 | 0.2 | 100 |
| Pc-2 | 0.2 | 0.1 | 0.3 | - | - | 98.8 | 0.2 | 0.2 | 0.2 | - | - | 100 |

It was found from the results of Table 1 that the Pc-1 contained 94.6% of the compound represented by the following formula (I-1-2) and 2.8% of the compound represented by the following formula (IA-1-2). It was also found that the Pc-2 contained 98.8% of the compound represented by the following formula (I-1-2).

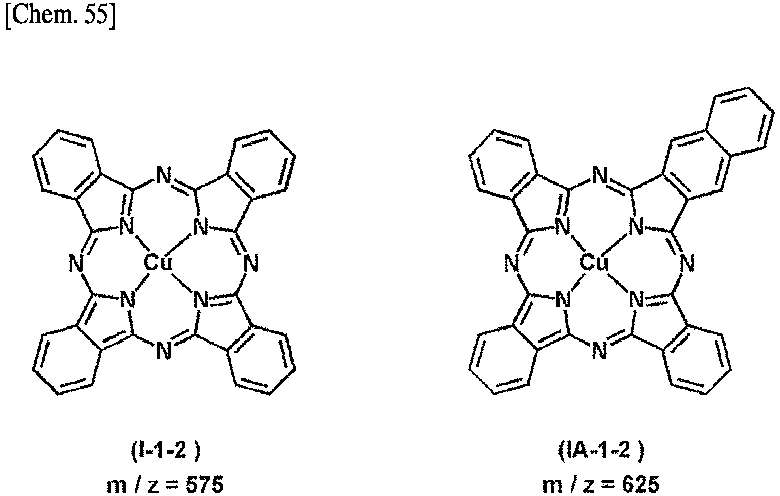

### (X-Ray Crystal Structure Analysis)

The crystal structures of the Pc-1 and Pc-2 were analyzed by X-ray diffraction (XRD), and then, it was found that the Pc-1 and Pc-2 had a crystal structure of *β*-type phthalocyanine.

### (TEM Observation)

The Pc-1, Pc-2, and RPc-1 obtained by Examples and Comparative Example and pigmented products thereof were subjected to TEM observation before and after pigmentation to check the state of the particles.

Here, pigmentation refers to an operation in which the crude product is further refined to a size that is suitable for practical use, and was performed as follows in Examples herein.

The crude product (0.5 g) was dispersed in diethylene glycol (0.6 g) together with sodium chloride (1.5 g), was pulverized with a Hoover Muller, and was dispersed in water. Then, the dispersion was filtered and was dried overnight with an oven at 98°C to prepare pigmented particles.

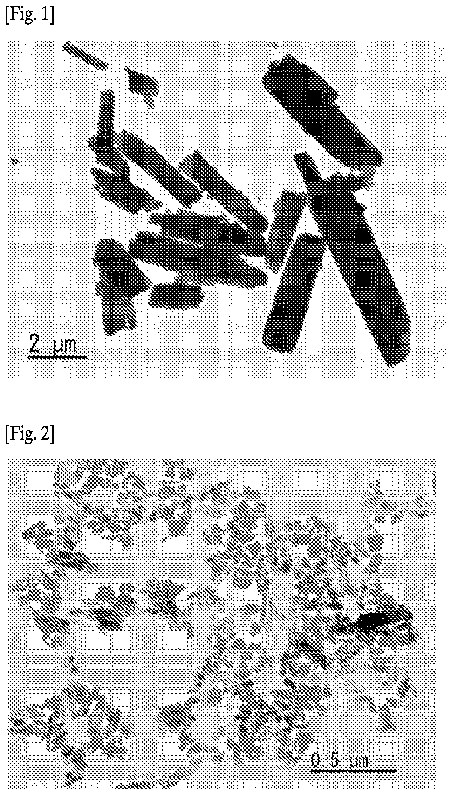

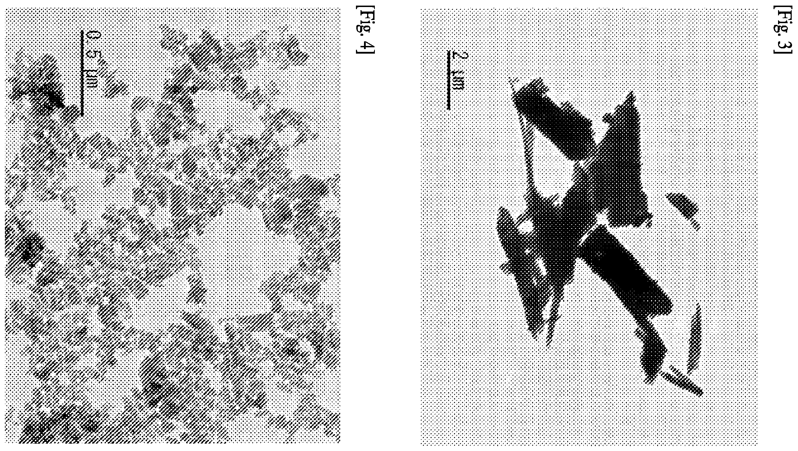

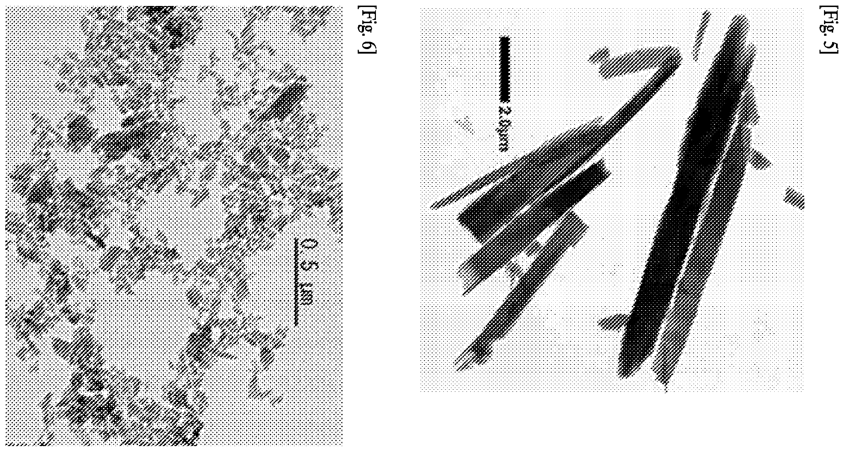

The results of TEM observation are shown in Figs. 1 to 6.

It was found from the TEM observation results of Figs. 1 to 6 that, in the copper phthalocyanine compounds of Examples of the invention, the particle size after pigmentation can be reduced as compared with the copper phthalocyanine of Comparative Example, and thus, the dispersibility in a resin and the brightness can be enhanced, leading to an increase in performance.

Since furan used in Synthetic Example 1 is derived from biomass, the phthalocyanine compounds of Examples of the invention can have an increased biomass degree. In addition, by incorporating substituents into furan as the raw material used in Synthetic Example 1, it is possible to produce phthalocyanine while controlling the positions and number of the substituents.

## Claims

1. A compound selected from a group of compounds represented by the following formulae (I), (II), (IA), (IIA), (IB), (IIB), (IC), (IIC), (ID), (IID), (IE), and (IIE), the compound containing a radioactive carbon atom ¹⁴C: 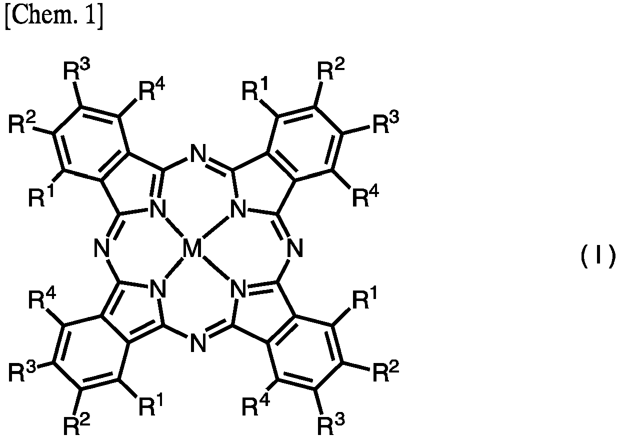 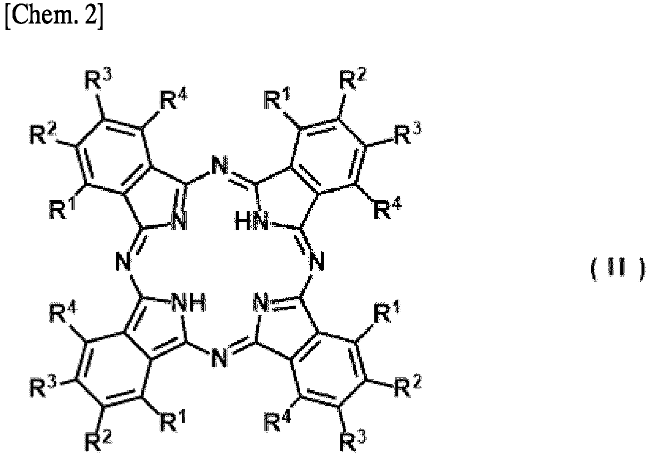 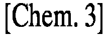 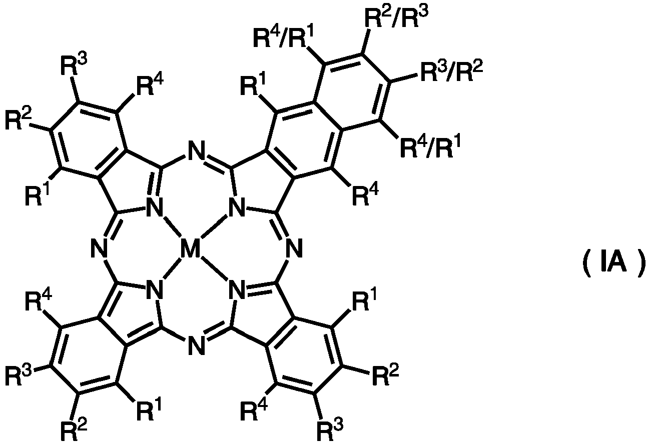 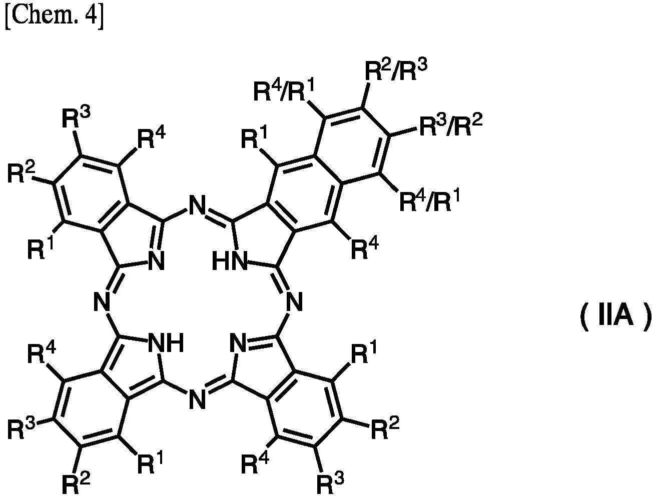 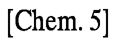 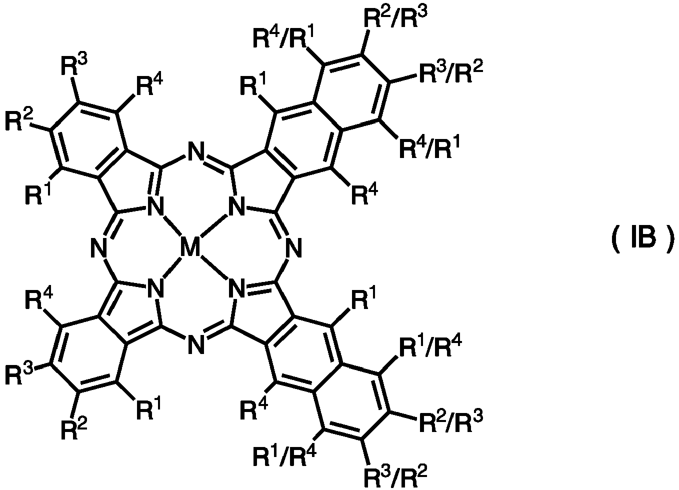 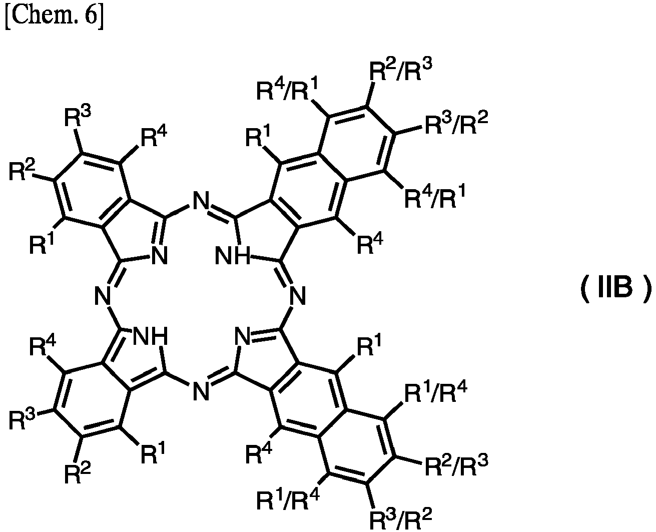 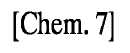 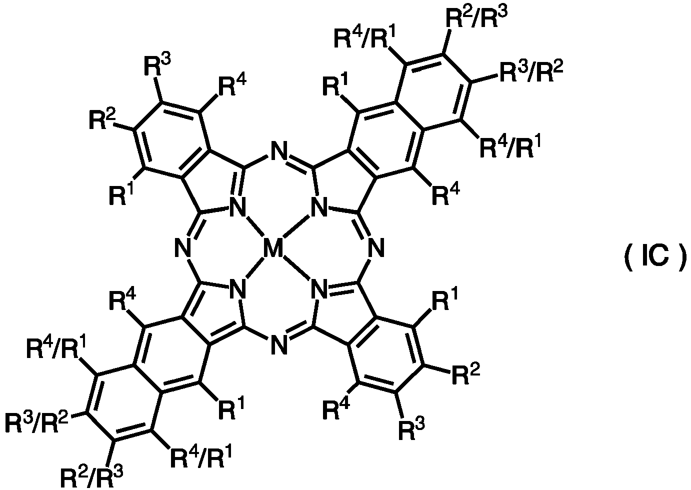 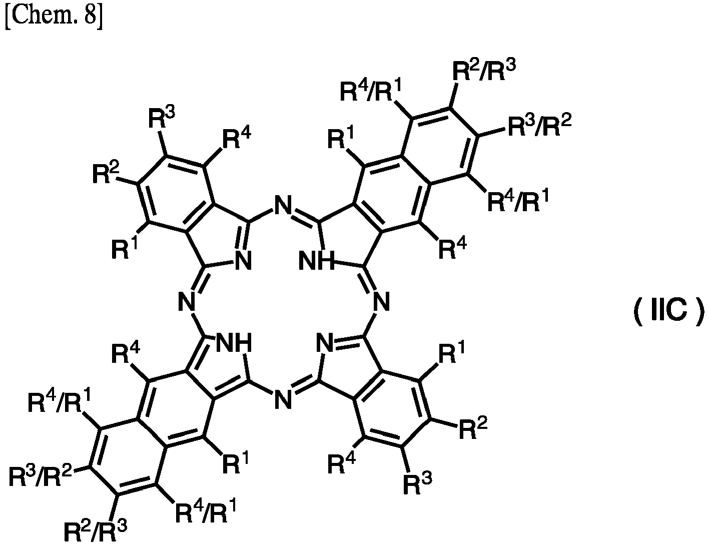 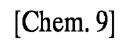 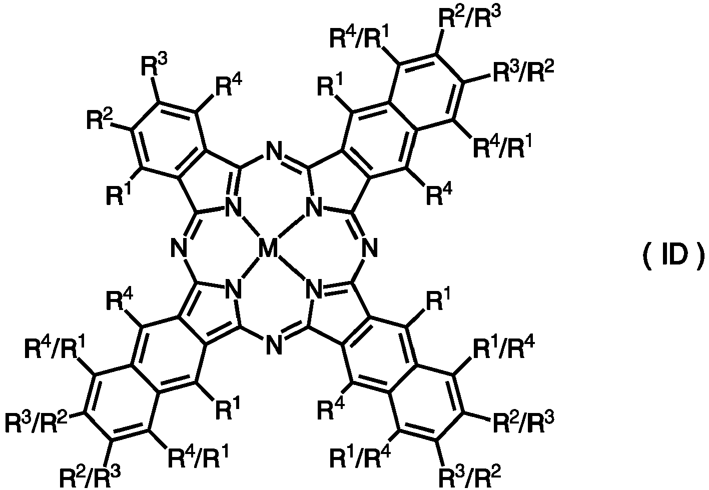 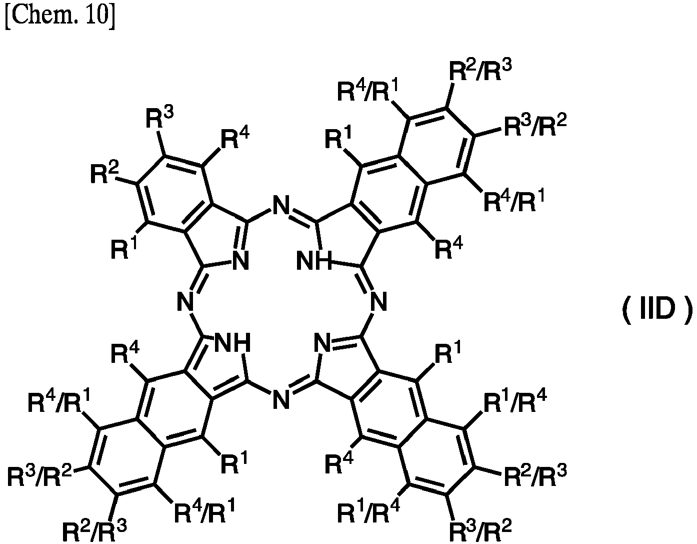 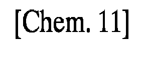 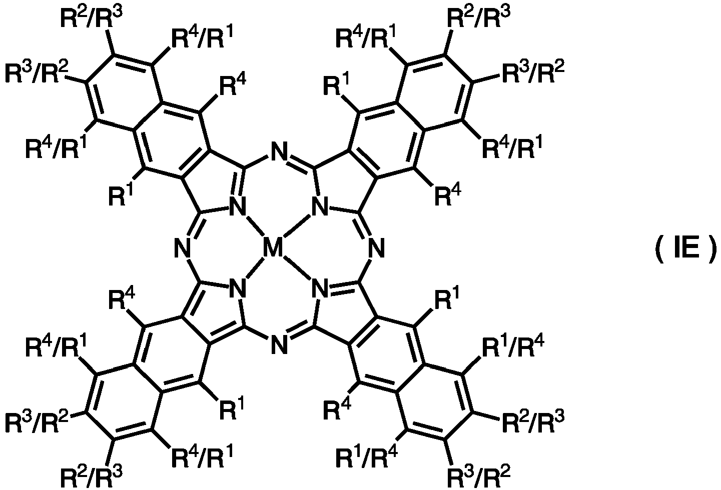 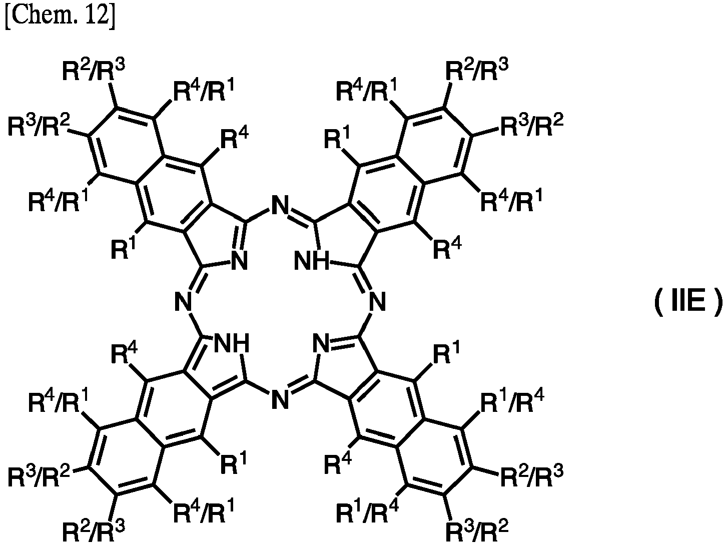 wherein in the formulae (I), (II), (IA), (IIA), (IB), (IIB), (IC), (IIC), (ID), (IID), (IE), and (IIE), R¹ to R⁴ each independently represent a hydrogen atom, a linear or branched alkyl group having 1 to 12 carbon atoms, a fluorine atom, a chlorine atom, a bromine atom, an iodine atom, a cyclohexyl group, or a phenyl group, one -CH₂- or non-adjacent two or more-CH₂- present in the alkyl group may be replaced by -C≡C-, -CH=CH-, -O-, -S-, -COO-,-OCO-, or -CO-, a hydrogen atom present in these groups may be substituted with a fluorine atom or a phenyl group, one -CH₂- or non-adjacent two or more -CH₂- present in the cyclohexyl group may be replaced by -O- or -S-, one -CH= or non-adjacent two or more-CH= present in the phenyl group may be replaced by -N=, one hydrogen atom or two or more hydrogen atoms present in this group may be substituted with a fluorine atom, and M represents a metal atom.

2. A compound according to claim 1, wherein the metal atom represented by M is one kind or two or more kinds selected from the group consisting of Al, Si, Sc, Ti, V, Mg, Fe, Co, Ni, Cu, Zn, Ga, Ge, Y, Zr, Nb, In, Sn, and Pb.

3. The compound according to claim 1 or 2, wherein the compound represented by (I), (II), (IA), (IIA), (IB), (IIB), (IC), (IIC), (ID), (IID), (IE), or (IIE) has pMC (percent modern carbon) of 20% or more.

4. The compound according to any one of claims 1 to 3, wherein R¹ to R⁴ are all a hydrogen atom.

5. A composition comprising one compound or two or more compounds selected from the group of compounds represented by the formulae (I), (II), (IA), (IIA), (IB), (IIB), (IC), (IIC), (ID), (IID), (IE), and (IIE) according to claim 1.

6. A pigment composition comprising the compound according to claim 1 or the composition according to claim 5.

7. A printing ink comprising the pigment composition according to claim 6.

8. A printed article comprising the printing ink according to claim 7 printed thereon.

9. A laminate comprising the printed article according to claim 8.

10. A paint comprising the pigment composition according to claim 6.

11. A painted article comprising a coating film formed of the paint according to claim 10.

12. A coloring composition for a color filter, the coloring composition comprising the pigment composition according to claim 6.

13. A color filter comprising the coloring composition for a color filter according to claim 12.
